# EUROPEAN PATENT APPLICATION

(11) **EP 4 696 350 A2**
(43) Date of publication of application: **18.02.2026**
(21) Application number: 25225210.1
(22) Date of filing: 23.12.2024
(51) Int. Cl.: A61M 15/00

(54) **COLLECTOR FOR APPARATUS FOR ADMINISTERING A MEDICAMENT TO A PATIENT**

(30) Priority: 10.01.2024 WO PCT/IB2024/000003
(62) Divisional of application: 24827848.3
(71) Applicant: Emphasys Importadora Exportadora e Distribuidora Ltda., 18540-000 Porto Feliz SP (BR)
(72) Inventor: Davies, Michael Birsha, Ceredigion, SA45 9 SW (GB); Tansley, Robert William, Leamington Spa Warwickshire, CV31 3JH (GB)
(74) Representative: DREISS Patentanwälte PartG mbB

(57) **Abstract**

There is provided an apparatus (100) for administering a medicament to a patient, comprising: a plurality of elongated medicament carriers, wherein closed pockets of the medicament carriers contain a medicament in powder form; and a dispensing mechanism configured to act on the plurality of elongated medicament carriers upon an actuation of an manual actuation lever (402) to dispense the medicament carried by the plurality of elongated medicament carriers.

## Description

### TECHNICAL FIELD

The invention relates to collectors for dry powder inhalers for administering at least one medicament to a patient.

### BACKGROUND OF THE INVENTION

US 8,161,968 B2 discloses a medicament dispenser for containing plural elongate form medicament carriers. A dispensing mechanism comprises at least one indexer for individually indexing the distinct medicament dose portions of each of the plural medicament carriers.

US 2023/381432 A1 discloses a dry powder inhaler for delivering medicament from at least one blister pack, each blister pack having a plurality of spaced-apart blister pockets containing doses of the medicament. The inhaler comprises: a housing for accommodating unused and used portions of the at least one blister pack together with a dispensing mechanism for simultaneously opening at least two blister pockets at a time; and a manifold component through which air can be drawn in use of the inhaler. The manifold component comprises: first and second air inlet openings for receiving external air a first air outlet opening for providing the external air to a first opened blister pocket and a first medicament inlet opening for receiving air-entrained medicament from the first opened blister pocket, the first air outlet opening and the first medicament inlet opening being arranged side-by-side to enable simultaneous communication with the first opened blister pocket; a second air outlet opening for providing the external air to a second opened blister pocket and a second medicament inlet opening for receiving air-entrained medicament from the second opened blister pocket, the second air outlet opening and the second medicament inlet opening being arranged side-by-side to enable simultaneous communication with the second opened blister pocket; and a medicament outlet opening for delivery of the air-entrained medicament from the first and second opened blister pockets to the user, the first and second medicament inlet openings being fluidly connected to the medicament outlet opening by a medicament delivery conduit formed in the manifold component. The first and second air inlet openings are fluidly connected to the first and second air outlet openings by respective first and second air conduits in the manifold component, wherein the air conduits are separately provided so that the external air from each of the first and second air inlet openings does not mix with the external air from the other of the first and second air inlet openings before reaching the first and second opened blister pockets.

US 2014/326239 A1 discloses a manifold for use in a medicament dispenser device for the delivery of medicament powder from an open blister pocket of a blister pack comprises a body defining a chimney having a chimney inlet and a chimney exit, and defining a chamber having a chamber inlet and a chamber exit, wherein the chimney exit and said chamber inlet lie side-by-side such that when the open blister pocket is positioned adjacent thereto airflow is directed from the chimney exit to the chamber inlet via the open blister pocket, and wherein one or more bleed holes are provided between the chimney and the chamber such that bleed airflow is able to be directed into the chamber to disruptively impact the airflow that transports the entrained medicament powder.

### SUMMARY OF THE INVENTION

The problems of the prior art are solved by a collector for an apparatus for administering a drug to a patient according to claim 1.

An aspect of the description is directed to the following subject matter: An apparatus for administering a medicament to a patient comprising: a plurality of elongated medicament carriers, each medicament carrier comprising a plurality of closed pockets extending along an imaginary curved line, wherein the closed pockets contain a medicament dose in powder form; at least one sealed pocket chamber configured to accommodate free-ended coiled-up portions of the plurality of elongated medicament carriers, the free-ended coiled-up portions comprising the respective plurality of closed pockets, wherein imaginary centre planes spanned by the respective imaginary curved line following the extension of the of the associated free-ended coiled-up portions are spaced apart at least in the area of the at least one sealed pocket chamber; and a dispensing mechanism configured to act on the plurality of elongated medicament carriers upon an actuation of a manual actuation lever to dispense at least one of the medicament doses carried by the plurality of elongated medicament carriers.

By stacking the plurality of medicament carriers, the construction of the dispensing mechanism offers several advantages. For example, the stacking allows using single parts or mechanisms that are shared between the elongated medicament carriers. In other words, the stacked medicament carriers enable a reduction in parts, thereby reducing the environmental footprint of the apparatus. In other words, the medicament carriers are stacked on top of each other in order to share one set of drive components. Furthermore, manufacturing of the apparatus benefits from the reduced number of parts and the resulting less complex construction.

A parallel alignment of imaginary centre planes allows stacking the plurality of medicament carriers on each other in a space-saving manner.

By reducing complexity and number of parts, environmental benefits are achieved. In particular, less polymers, fewer types of polymers and no metal parts are necessary to provide the apparatus.

An advantageous example is characterized in that separate neighbouring sealed pocket chambers for accommodating a respective one of the free-ended coiled-up portions are separated by a separation wall, wherein the separated sealed pocket chambers are configured to accommodate at least the free-ended coiled-up portion of the respective one of the plurality of elongated medicament carriers.

By providing a separation wall, the medicament carriers do not interfere with each other when being transported or moved.

An advantageous example is characterized in that the dispensing mechanism comprises a transport mechanism configured to pull a plurality of peeled-off lid foils causing a transport of at least one opened pocket of an associated base foil of the plurality of medicament carriers.

Advantageously, a shared transport mechanism reduces the number of parts. Moreover, additional indexing wheels, further gear constructions going along with advanced material requirements are avoided by pulling the peeled-off lid foil in order to transport the opened pocket.

An advantageous example is characterized in that the at least one transport mechanism comprises a plurality of transport hubs, each with an attachment portion configured to secure a leading portion of the lid foil, the transport hubs are rotatable about an actuation axis.

The transport hubs are therefore configured for opening and transporting the opened pockets towards the collector.

An advantageous example is characterized in that the at least one transport mechanism comprises a transport shaft to which the plurality of transport hubs are attached.

Advantageously, the shared transport shaft further reduces the necessary number of parts. Furthermore, complex gears are avoided in favour of the common or shared transport shaft.

An advantageous example is characterized in that the at least one dispensing mechanism comprises the manual actuation lever that is configured to provide a torque to the transport mechanism, especially configured to provide the manual torque to the transport shaft.

An advantageous example is characterized in that the manual actuation lever is a movable cover configured to be rotatable about the actuation axis and configured to cover a mouthpiece in a first state and to uncover the mouthpiece in a second state.

An advantageous example is characterized in that the at least one transport mechanism comprises a foil rewind prevention mechanism that is arranged between the manual actuation lever and the remaining part of the transport mechanism, especially between the manual actuation lever and the transport shaft.

Advantageously, the foil rewind prevention mechanism preserves the actuation direction and the transmission of torque towards the transport hubs only in the actuation direction. Therefore, the foil rewind prevention mechanism ensures that the lid foils are coiled up on the transport hubs.

An advantageous example is characterized in that the foil rewind prevention mechanism is configured to transfer the torque in an actuation direction of rotation to the plurality of transport hubs and configured to prevent torque from being transferred to the transport hubs in the opposite direction of the actuation direction of rotation.

Advantageously, the foil rewind prevention mechanism applies the torque in only one direction of movement, the actuation direction, to the hubs. This simplifies the design of the transport mechanism towards the hubs.

An advantageous example is characterized in that the foil rewind prevention mechanism comprise: A ratchet wheel with a plurality of teeth, each one with a sliding portion and an engagement portion, the plurality of teeth arranged along an imaginary circle around the actuation axis; and a pawl with at least one arm rotatable about the actuation axis, the at least one flexible arm configured to pass the sliding portion in order to and configured to engage with the engagement portions in order to transfer the torque to the transport shaft.

The ratchet wheel and the pawl provide a robust and compact solution.

An advantageous example is characterized in that an actuator rotation limiting mechanism comprises first limiting means configured to delimit a rotational movement of the manual actuation lever to a first angular range; the plurality of teeth configured to delimit a rotational movement of the transport mechanism, especially of the shaft, to a second angular range.

Advantageously, the adaption of the angular ranges ensures that at least one opened pocket arrives at position with respect to the collector, so that an air-flux can pass the pocket by means of the collector. Moreover, these fixed angular ranges further ensure that the user gets a feedback about what one actuation means, namely a single rotation of the mouthpiece cover towards its opened position.

An advantageous example is characterized in that the dispensing mechanism comprises: at least one peeling edge configured to peel off the lid foil of a respective one of the plurality of medicament carriers from the base foil of the respective one of the plurality of medicament carriers thereby uncovering an opening of at least one pocket in the respective base foil, the medicament is accommodated in the respective opened pocket.

Advantageously, the peeling edge enables the apparatus to peel of the lid foil from the base foil only for that dose that is intended to be applied in a single actuation of the apparatus. The remaining pockets for further actuations remain closed.

An advantageous example is characterized in that the dispensing mechanism comprises a collector configured to collect the medicament from an opened pocket of the each base foil via an airflow that passes through the respective pocket and configured to deliver the resulting medicament-air mixture towards the mouthpiece.

Therefore, the shared collector provides to collect medicament doses from two different pockets, thereby enabling the patient to inhale a medicament-air-mixture consisting of two different types of medicaments.

An advantageous example is characterized in that the collector comprises at least one port section interrupted at least by a plurality of pairs of at least one inlet port and at least one outlet port, wherein the pairs are arranged such that, in use, underpressure on the side of the corresponding outlet port causes air to be sucked through the inlet port, the air passing through the open pocket thereby creating a medicament-air-mixture, and the medicament-air-mixture exiting through the outlet port towards the mouthpiece.

An advantageous example is characterized in that the plurality of pairs, especially the respective inlet and outlet ports, are arranged along a collector axis that is parallel to the actuation axis.

Advantageously, the common collector axis enables the desired stacked arrangement of the medicament carriers.

An advantageous example is characterized in that the at least one port section comprises at least one concave or convex surface especially extending parallel to the collector axis.

Advantageously, the concave or convex guiding surface follows the pre-bent form of the medicament carrier in the sealed pocket chamber. This measure allow a wider tolerance with respect to alignment of pockets to the collector. Therefore, possible wider tolerances are compensated and the quality for the medicament dose delivery is assured.

When the patient starts inhaling, the resulting underpressure causes the base foil, which surrounds the opening of the pocket, to adhere to the guiding surface of the collector. Therefore, airflow between the base foil and the collector guiding surface is reduced and an airflow from a collector inlet, through the pocket, and exiting the pocket through a collector outlet is increased. Therefore, the increased or wider tolerances in particular with regard to the pocket position are compensated and the quality of the medicament dose delivery is increased.

In the prior art, the requirement of a precise pocket alignment is often overestimated. Instead, tolerances with respect to the pocket alignment can be accepted up to a certain extent. Furthermore, this description provides several counter-measures like the bent contour of the collector surface surrounding collector inlets and outlets to compensate the accepted wider tolerances.

An advantageous example is characterized in that the transport mechanism comprises a plurality of base winder sections, each base winder section comprising an attachment portion for securing a leading portion of an associated base foil of the plurality of medicament carriers; the base winder sections are rotatable about a wind-up axis.

Advantageously, the wind-up sections ensure that the waste base foil is safely discharged from the area of the collector. The wind-up sections ensure that the base foils are wound up in a corresponding wind-up chamber, which means that the entire apparatus can be smaller.

An advantageous example is characterized in that a wind-up shaft provides the plurality of wind-up sections.

By providing a single wind-up shaft, the number of parts is further reduced.

An advantageous example is characterized in that the base winder sections and the transport shaft are motion-mechanically connected with each other.

This ensures that both the base winder sections and the transport shaft act in a synchronized manner in order to safely transport the coiled part of the medicament carrier with closed pockets and to coil the waste part of the base part with the empty pockets.

An advantageous example is characterized in that at least one gear, especially a single units gear, meshes with a drive gear connected to the transport shaft.

Advantageously, a simple constructions is provided to pass on torque to the at least one gear. In particular, the single units gear meshes directly with the drive gear and therefore provides a simple solution for informing the user about the already used doses or remaining doses.

An advantageous example is characterized in that the at least one gear further meshes with a base winder gear connected to the base winder shaft.

Advantageously, the based winder gear is driven by the at least one gear or only a single gear arranged between the drive gear and the base winder gear. Therefore, a constructional simplification is achieved.

An advantageous example is characterized in that the at least one gear and the base winder gear comprise a human readable numbering representing the numbers of doses that have been already consumed with the apparatus.

Advantageously, the gears provide two functions: transmission of a torque to the base winder gear and information provision to the patient about the remaining or used medicament doses.

According to an aspect of the description, there is described an apparatus for administering a medicament to a patient, the apparatus comprising: at least one elongated medicament carrier, wherein the at least one medicament carrier comprises a plurality of closed pockets that extend along the at least one medicament carrier, wherein the closed pockets contain a medicament in powder form; a mouthpiece with an outer opening; at least one collector with at least one conduit, wherein the at least one conduit extends extends along a longitudinal axis through the collector from at least one entry section toward the opening of the mouthpiece; wherein the entry section of the conduit comprises: at least one collector window over which the respective opened pocket is moved, the at least one collector window comprising at least one pocket airflow entry and at least one pocket airflow exit, wherein the pocket airflow entry is configured to receive air to enter an opened pocket of an elongated medicament carrier arranged at the at least one collector window, wherein the at least one pocket airflow exit has at least one pocket-side opening with its imaginary normal axis oriented at an angle that is generally orthogonal to the longitudinal axis of the conduit, with a variation of up to ±45°, in particular ±35°, in particular ±10°, from the orthogonal alignment between the normal axis N and the longitudinal axis.

Advantageously, the arrangement of the conduit and the at least one opening of the pocket airflow exit provide that the opened pockets - that adhere to the surrounding collector window, when negative pressure is applied - are arranged horizontally during the use of the apparatus. The patient will therefore automatically try to orientate the whole apparatus into a position in which the opening of the pockets are arranged horizontally. This arrangements avoids that the powder in the opened pockets falls out. Further structures that avoid that the powder falls out of the pocket can be minimized.

In other words, the spatial alignment of the pocket-side opening of the pocket airflow exit and the at least one conduit that therefore the mouthpiece encourages the patient to maintain the opened pocket below the opening in a more or less horizontal position. This horizontal position is beneficial for the discharge of the powder out of the opened at least one pocket.

According to an example, a pair of parallel imaginary inner planes extend through innermost portions of the inner wall of the at least one conduit, wherein the at least one pocket airflow exit has the at least one pocket-side opening with its imaginary normal axis being orthogonal to both parallel imaginary inner planes.

According to an example, the mouthpiece has an outer contour with a first extension, that extends orthogonal to the imaginary normal axis of the at least one airflow exit, that is greater than a second extension, that extends parallel to imaginary normal axis of the at least one airflow exit.

Advantageously, the form of the mouthpiece indicates that the user moves the apparatus such that the greater extension of the mouthpiece is arranged horizontally to match the shape of their mouth.

According to another example, an outer surface of a body of the apparatus comprises a tapering section, wherein the tapering section tapers at least on one dimension orthogonal to the at least one normal axis of the opening of the at least one pocket airflow exit toward a distal end of the body that faces away from an actuation lever.

Advantageously, the handling of the apparatus is self-explaining by providing the tapering section that the patient will try to grasp with one hand. With his other hand the patient will grasp the movable cover or actuation lever. In other words, the apparatus will be brought into an upright position by arranging the tapering section along the normal axis of the opening of the at least one pocket airflow exit.

According to an example, the conduit includes a bend in its longitudinal path at an intermediate portion between the opening of the mouthpiece and the at least one pocket airflow exit, wherein said bend changes the direction of the conduit, and wherein said bend directs away from a side of the conduit containing the opening of the at least one pocket airflow exit.

Advantageously, the bend enables improved haptics for use, as the bend allows the mouthpiece and the part of the conduit directly behind it to be orientated towards the mouth of the patient.

According to another example, said bend alters the direction of the conduit by an angle between 120 degrees and 178 degrees, specifically between 160 degrees and 175 degrees, specifically between 170 and 175 degrees, relative to the longitudinal path of the conduit 440 before and after the bend.

The ranges for said bend are beneficial for orienting the apparatus in the desired position.

According to a further example, the at least one pocket-side opening of the at least one pocket airflow exit is oriented at an angle of substantially 90 degrees relative to the longitudinal path of a straight section of the conduit preceding the bend.

Advantageously, the pocket airflow exit will be oriented horizontally by the patient before opening and inhaling the medicament-air mixture.

According to a further example, the mouthpiece in fluid connection with the collector window, wherein the mouthpiece is configured to allow the patient to inhale a medicament-air mixture that comprises at least partly the medicament dose from the opened pocket arranged at the collector window.

According to a further example, the apparatus comprises: a peeling edge configured, together with a manual actuation lever and a transporter hub, to peel off the lid foil of the at least one medicament carrier from the base foil thereby opening the at least one of the plurality of closed pockets in the base foil; the transporter hub that is operated by the manual actuation lever and that is rigidly connected to the transporter gear, wherein the transporter hub comprises an attachment portion for securing a leading portion of the lid foil, and wherein the transporter hub is configured, together with the peeling edge, to move the opened pocket of the base foil toward the collector window solely via a tensile force coupled into the lid foil by means of the transporter hub; and a foil rewind prevention mechanism configured to prevent an unintended rewind of the lid foil.

An aspect of the description is directed to the following subject matter: A collector for an apparatus for administering a medicament to a patient the collector comprising: at least one conduit extending through the collector from at least one an entry section toward a mouthpiece; wherein the entry section of the conduit comprises: at least one collector window with at least one pocket airflow entry and at least one pocket airflow exit, the pocket airflow entry configured to receive air to enter an opened pocket of an elongated medicament carrier arranged at the at least one collector window, the at least one pocket airflow exit configured to receive a medicament-air mixture from the opened pocket to enter the at least one conduit; and at least one bypass port configured to receive air to enter the at least one conduit, wherein a distance between the at least one bypass port and the at least one pocket airflow exit is at least greater than an equivalent diameter of an imaginary circular opening having the same cross-sectional area as the associated at least one pocket airflow exit and/or wherein the at least one bypass port and the at least one pocket airflow exit lead into opposing sides of the at least one conduit.

As the bypass port is on the opposite side from the pocket airflow exit and/or both openings have the minimum distance between each other, fluid flow inside the conduit is influenced more effectively by the pressure difference between the atmospheric pressure at the bypass and the negative pressure at the mouthpiece. This configuration encourages smoother and more consistent airflow from the outside into the conduit, leveraging the pressure gradient across the system.

Compared with the prior art: When the bypass opening and the pocket airflow exit are adjacent, air entering the collector may not be as efficiently directed or managed due to turbulence created by the proximity of airflows. This can lead to lower overall performance as the flows interfere with each other, reducing the system's ability to take full advantage of the pressure differentials.

By placing the bypass port and the pocket airflow exit on opposing sides and/or by maintaining the minimum distance, the structure minimizes interference between the two flows. Air entering from the bypass and pocket airflow exit moves through the channel in a more streamlined manner, avoiding turbulence and pressure backflows.

Compared with prior art: When the air inlet and bypass opening are next to each other, there is a higher airflow interference. The medicament-air mixture mixes with air from the bypass port, creating turbulence or chaotic flow patterns that reduce efficiency. This leads to suboptimal performance in terms of maintaining consistent air pressure and velocity through the system.

In the proposed design where the openings are on opposite sides, the pressure drop across the collector is better managed. Air moves smoothly from high to low pressure, facilitating efficient air transfer and minimizing losses.

Compared with prior art: Placing the air inlet and bypass together causes local pressure imbalances, leading to inconsistent pressure drops. This reduces the effectiveness of the collector's ability to transfer the medicament-air mixture efficiently to the mouthpiece.

The airflow generated in the proposed collector has a longer and more distinct path through the conduit when the openings are on opposite sides or maintain the minimum distance. This allows for better mixing of the air and medicament air-mixture entering from different sources, ensuring a uniform flow into the conduit.

Compared with prior art: If the bypass and air inlet are adjacent, the mixing and flow paths become more convoluted, leading to flow recirculation and loss of energy, which decreases air performance.

The proposed collector advantageously provides a good medicament delivery performance over a wide range of applied negative pressure at the mouthpiece that varies from patient to patient.

An advantageous example is characterized in that the at least one conduit comprises at least one diffusor section along an air path from the entry section towards the mouthpiece.

The diffusor section provides the necessary turbulences in order to mix the airflow with the medicament-air flow.

An advantageous example is characterized in that the diameter increase of the at least one diffusor section is implemented along an imaginary plane arranged between the at least one bypass port and at least one pocket airflow exit.

The diameter increase towards the mouthpiece that is mainly provided in said imaginary plane allows homogenizing the medicament-air mixture while the height of the collector that runs perpendicularly to the imaginary plane may remain constant.

An advantageous example is characterized in that the conduit comprises a curvature or bend along its length, which defines an angle of less than 180° and more than 110°, in particular less than 180° and more than 150°, measured in a direction facing away from the at least one open pocket.

Advantageously, the opened pocket is arranged in a horizontal manner to avoid that the powder falls out of the pocket. The curvature or bend allows arranging the mouthpiece in an inclined angle facing upwards in the direction of the patient's mouth.

An advantageous example is characterized in that an open cross-section of the at least one bypass port is equal or greater than an open cross section of the associated at least one pocket airflow entry or at least one pocket airflow exit.

Advantageously, the amount of air entering the bypass port is greater than or equal to the amount of medicament-air mixture entering the conduit via the pocket airflow exit. Therefore, the bypass air that enters the conduit provides a suction effect for the medicament-air mixture flow.

An advantageous example is characterized in that a distance between the at least one bypass port and the at least one pocket airflow exit is equal or greater than 1.4 multiplied with the equivalent diameter of an imaginary circular opening having the same cross-sectional area as the associated at least one pocket airflow exit.

Advantageously, the minimum distance ensures good medicament delivery performance due to the separated fluid flows in the entry section.

An advantageous example is characterized in that the at least one bypass port and the at least one pocket airflow exit extend parallel to each other.

Therefore, the entry angle of the air and the air-medicament mixture into the conduit is similar.

An advantageous example is characterized in that the apparatus comprising: the at least one elongated medicament carrier, the at least one medicament carrier comprises a plurality of closed pockets that extend along the at least one medicament carrier, wherein the closed pockets contain a medicament in powder form, wherein the closed pockets are formed by recesses in a base foil, wherein a lid foil closes the plurality of openings of the recesses in the base foil; a peeling edge configured, together with a manual actuation lever and a transporter hub, to peel off the lid foil of the at least one medicament carrier from the base foil thereby opening the at least one of the plurality of closed pockets in the base foil; the manual actuation lever; the transporter hub that is operated by the manual actuation lever, the transporter hub comprises an attachment portion for securing a leading portion of the lid foil, wherein the transporter hub is configured, together with the peeling edge, to move the opened pocket of the base foil toward a the collector window solely via a tensile force coupled into the lid foil by means of the transporter hub; a foil rewind prevention mechanism configured to prevent an unintended rewind of the lid foil; the collector window, over which the respective opened pocket is moved; and the mouthpiece in fluid connection with the collector window by the conduit. The mouthpiece is configured to allow the patient to inhale a medicament-air mixture comprising powder from the at least one opened pocket arranged at the collector window.

Another aspect of the description is directed to the following subject matter: a dose counter for an apparatus for administering at least one medicament to a patient the dose counter comprising: a manual actuation lever; a transporter gear that is operated by the manual actuation lever, wherein the transporter gear is configured to rotate a certain actuation angle for each actuation of a dose delivery of the at least one medicament, wherein each actuation opens at least one pocket of an elongated medicament carrier, wherein the at least one opened pocket contains the at least one medicament and allows an airflow to expel the at least one medicament from the at least one opened pocket; a ones gear engaging with the transporter gear and configured to rotate one tenth of a turn for each actuation of the transporter gear, wherein the ones gear includes a section that comprises human-readable numerals from 0 to 9 along an imaginary circular line; a tens disc rotatably mounted, wherein the tens disc is configured to rotate a certain angle upon each passage of the numeral 0 on the ones gear to indicate the next decimal place of the count, wherein the ones gear includes at least two numerals along an imaginary circular line, wherein the most neighbouring numerals displayed by the ones gear and the tens disc together represent the number of remaining or used doses of the at least one medicament; and an engagement mechanism between the ones gear and the tens disc configured to rotate the tens disc upon or after the passage of the numeral 0 on the ones gear.

The dose counter provides the following advantages.

Accurate Dose Tracking: The combination of the ones gear and tens disc provides a precise and clear indication of the remaining or used doses, ensuring that the user can easily track how many doses are left. This helps avoid under- or overuse of the medicament, improving treatment adherence and safety.

Sequential and Logical Counting: By using a tens disc and ones gear in an interlocked system, the mechanism follows a logical decimal counting method. Each time the ones gear completes a full rotation, the tens disc moves to indicate the next decimal value, providing an intuitive and familiar counting experience to users.

Mechanical Simplicity and Reliability: The mechanical interaction between the ones gear and the tens disc provides a robust and straightforward solution for counting doses without requiring complex electronics or sensors. This increases the reliability and durability of the device, which is particularly important in healthcare applications where consistent performance is critical.

Clear Visual Feedback: The alignment of the neighbouring numbers on the ones gear and tens disc clearly displays the remaining or used doses at a glance. This feature is user-friendly, especially for individuals with limited dexterity or vision impairments.

No Power Source Required: As a fully mechanical system, the dose counter does not require any electrical power, making the device more environmentally friendly, reducing manufacturing costs, and eliminating the risk of battery failure. This is particularly beneficial for devices that may be used over extended periods or in regions with limited access to power.

Scalability: The mechanism is easily adaptable to different devices and dosing regimens. The counting mechanism can be customized for various medicament types or delivery systems that require different numbers of doses.

Prevents Overuse: By clearly displaying the number of doses remaining, the mechanism helps prevent the user from attempting to use the device when the medicament is depleted. This ensures proper medication dosing and prevents ineffective usage of the device.

Cost-Effective Manufacturing: The mechanical design, with gears and discs, allows for cost-effective mass production using standard manufacturing processes. This simplicity contributes to a lower overall cost for the device, benefiting both manufacturers and users.

Enhanced User Experience: The visual and mechanical feedback from the dose counter enhances the user experience by providing immediate and clear information about the device's status, reducing the likelihood of confusion or misuse.

An advantageous example is characterized in that the ones gear is configured to display ones or units of the decimal number of actuations remaining, and the tens disc is configured to display the tens of the decimal number.

Advantageously, the ones gear and tens disc provide a mechanically simple mechanism to count and indicate the doses.

Advantageously, the tens disc remains in its position during the counting of the ones.

An advantageous example is characterized in that the tens disc comprises a plurality of recesses following an outward contour of the section of the ones gear.

An advantageous example is characterized in that the tens disc and the ones gear are geared such that the tens disc rotates by a predetermined angular increment, in particular one ninth of a turn, each time a section between numeral 0 and numeral 9 of the ones gear passes.

Advantageously, this mechanism provides the appropriate turning of the tens disc.

An advantageous example is characterized in that an outward contour of the ones gear is interrupted by a recess, wherein the recess engages with a respective projection of the tens disc, said projection arranged between neighbouring recesses of the tens disc.

An advantageous example is characterized in that a rotatable flag positioned to cover at least a portion of the ones gear and the tens disc; an opening in the rotatable flag configured to display the most neighbouring numerals of the ones gear and the tens disc; the rotatable flag is configured to rotate upon the engagement of a dog of the rotatable flag with the tens disc after the last pocket is opened, such that the opening in the flag is rotated away to obscure the dose counter.

Advantageously, the rotatable flag, for example in red, informs the user that the inhaler is near empty. With this information the user can readily get a new one in order to continue the prescribed treatment.

An advantageous example is characterized in that the engagement of the dog with the tens disc is triggered after the dose counter displays zero, and further actuation beyond this point causes the tens disc to turn and engage with the dog to rotate the rotatable flag.

An advantageous example is characterized in that the dog and the rotatable flag are configured to rotate the flag only after the final dose is expelled, ensuring that the dose counter is obscured only after the last use of the device.

An advantageous example is characterized in that the does counter 280 further comprises a base winder gear engaging with the ones gear and configured to rotate one a defined degree of a turn for each actuation of the transporter gear, wherein the base winder gear connected to a base winder shaft that comprises at least one wind-up section to wind-up a base foil.

An aspect of the description is directed to the following subject matter: An apparatus for administering a medicament to a patient the apparatus comprising the dose counter according to the second aspect.

An advantageous example is characterized in that the apparatus comprises: the at least one elongated medicament carrier, the at least one medicament carrier comprises a plurality of closed pockets that extend along the at least one medicament carrier, wherein the closed pockets contain a medicament dose in powder form, wherein the closed pockets are formed by recesses in a base foil, wherein a lid foil closes the plurality of openings of the recesses in the base foil; a peeling edge configured, together with the manual actuation lever and a transporter hub, to peel off the lid foil of the at least one medicament carrier from the base foil thereby opening the at least one of the plurality of closed pockets in the base foil; the transporter hub that is operated by the manual actuation lever and that is rigidly connected to the transporter gear, the transporter hub comprises an attachment portion for securing a leading portion of the lid foil, wherein the transporter hub is configured, together with the peeling edge, to move the opened pocket of the base foil toward the collector window solely via a tensile force coupled into the lid foil by means of the transporter hub; a foil rewind prevention mechanism configured to prevent an unintended rewind of the lid foil; a collector window, over which the respective opened pocket is moved; and a mouthpiece in fluid connection with the collector window, the mouthpiece is configured to allow the patient to inhale a medicament-air mixture comprising powder from the at least one opened pocket arranged at the collector window.

### BRIEF DESCRIPTION OF THE DRAWINGS

- Fig. 1: depicts an apparatus for administering a medicament to a patient in a closed state;
- Fig. 2: depicts the apparatus of figure 1 in an open state;
- Fig. 3: depicts the apparatus in an exploded view;
- Fig. 4: depicts two medicament carriers arranged parallel to each other in a perspective view;
- Fig. 5: depicts a detail of a dose counter in a perspective view;
- Fig. 6: depicts a collector of the apparatus in a perspective view;
- Fig. 7: depicts another detail of the one-strip apparatus in perspective view;
- Fig. 8: depicts a section through the apparatus;
- Fig. 9: depicts a flow diagram for manufacturing the apparatus;
- Figs. 10a-c: depict different alignment positions of pockets with respect to the collector;
- Fig. 11: depicts an airflow through inlets and outlets of collector windows and pockets;
- Fig. 12: depicts a collector in a cut along its conduit;
- Fig. 13: depicts a single-strip apparatus with only one medicament-carrier;
- Fig. 14: depicts an example of the collector for the apparatus of Fig. 13;
- Fig. 15: depicts a dose counter in an intermediate position indicating 30 remaining doses;
- Fig. 16: depicts the dose counter in a subsequent position indicating 29 remaining doses;
- Fig. 17: depicts the dose counter with a rotatable flag, the dose counter indicating 9 remaining doses;
- Fig. 18: depicts the dose counter indicating zero remaining doses;
- Fig. 19: depicts the dose counter indicating that the apparatus is empty;
- Fig. 20: depicts the dose counter with a winder gear visible; and
- Fig. 21: depicts a schematic flow diagram for operating the apparatus.

### DETAILED DESCRIPTION OF THE DRAWINGS

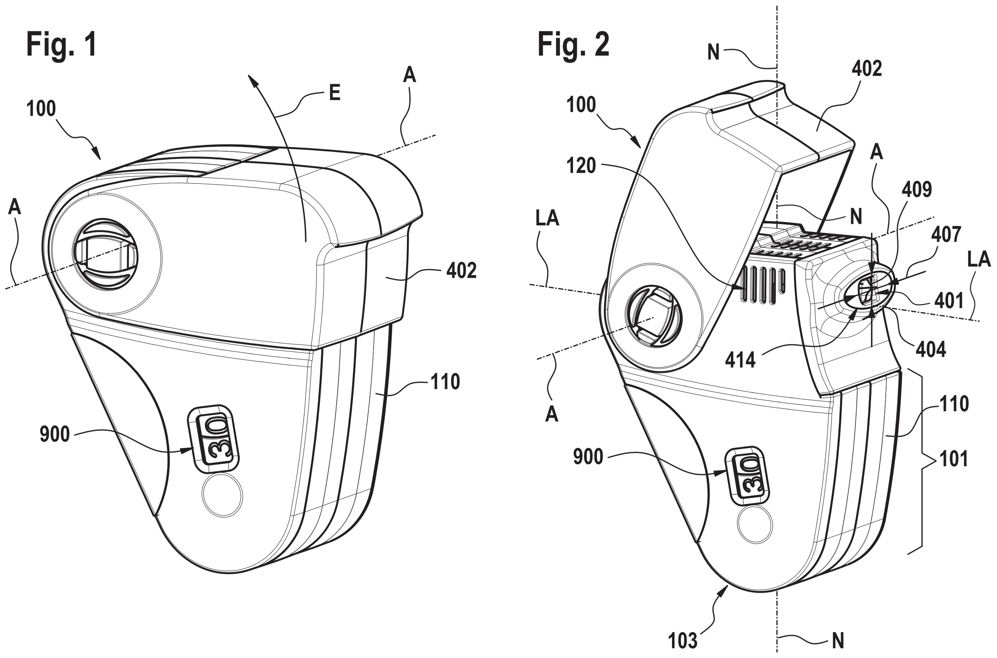

Figures 1 and 2 depict an apparatus 100 in a perspective view. The apparatus 100 or dry powder inhaler is configured for administering at least one medicament, in particular at least two different medicaments, to a patient. The apparatus 100 comprises a manual actuation lever 402 in form of a mouthpiece cover, the manual actuation lever 402 rotatable attached to a main body 110. A counting mechanism 900 is visible through an opening in the main body 110. The counting mechanism 900 is configured to display the individual doses of the drug that are still available. The manual actuation lever 402 is part of dispensing mechanism 400 that is configured to act on a plurality of elongated medicament carriers arranged inside the apparatus 100. Upon an actuation of the manual actuation lever 402, at least one of a plurality of medicament doses carried by the plurality of elongated medicament carriers is dispensed. The closed position of the apparatus 100 is shown in Figure 1. For dispensing the at least one medicament dose, the manual actuation lever 402 is rotated about an actuation axis A into a direction E of rotation from the closed position to an open position that is shown in figure 2.

In the open position, the apparatus 100 exposes a mouthpiece 404. At his point in time, the dispensing has internally provided the medicament ready to be inhaled by the patient by means of the mouthpiece 404. Additionally, the apparatus 100 uncovers external air inlets 120, through which, upon applied underpressure at the mouthpiece 404, air is drawn in through the air inlets 120, mixed with the powdered drug, and provided at the opening of the mouthpiece 404 as a medicament-air mixture for inhalation. In other words, in figure 2 the apparatus 100 is ready for dose delivery by inhaling via the mouthpiece 404. The main body 110 comprises at least a drive cover 112, a first component 114a, a second component 114b, and a strip cover 116.

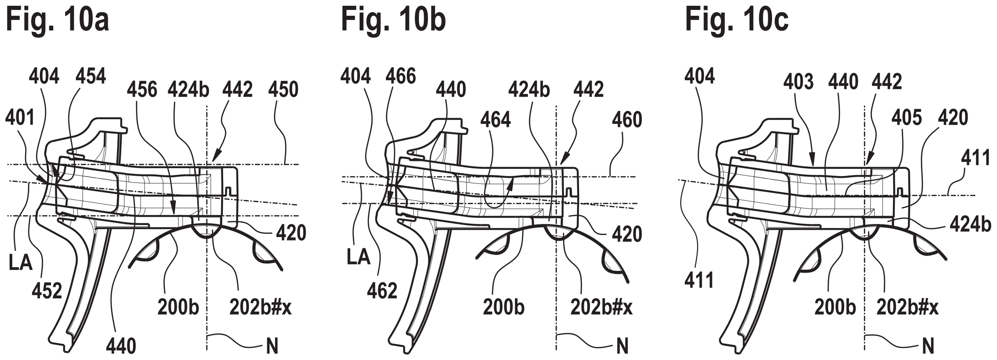

At least one pocket airflow exit, positioned within the apparatus 100, features at least one pocket-side opening aligned with an imaginary normal axis N. This arrangement is further elaborated upon with reference to figure 10a.

The mouthpiece 404 has an external contour 414, which includes a first extension 407 that extends orthogonally to the imaginary normal axis N of the at least one airflow exit 424a-b. This first extension 407 exceeds the length of a second extension 409, which extends parallel to the imaginary normal axis N of the at least one airflow exit 424a-b or which extends in an imaginary plane that runs through the normal axis N.

In other words: The at least one pocket airflow exit 424a-b includes at least one pocket-side opening whose imaginary normal axis N is oriented at an angle of 90° ±45° relative to the longitudinal axis LA of the conduit 440, preferably within ±35° (90°±35°), and more preferably within ±10° (90°±10°), and even more preferably within ±5° (90°±5°) of orthogonality. The same applies to the opening of the pocket that is arranged below the pocket airflow exit 424a-b.

Described differently: The at least one pocket airflow exit 424a-bincludes at least one pocket-side opening whose normal axis N that is oriented such that the angle between the longitudinal axis LA of the conduit 440 and a plane perpendicular to the normal axis N is within ±45°, preferably within ±35°, and more preferably within ±10°, and even more preferably within ±5°.

An outer surface of the apparatus body 110 incorporates a tapering section 101. This tapering section 101 narrows along at least one dimension orthogonal to the normal axis N of the opening of the pocket airflow exit 424a-b, converging towards a distal end 103 of the body 110. This distal end 103 is positioned away from the actuation lever 402, facilitating the patient's grip on the tapering section 101 with one hand, while enabling the other hand to operate the apparatus 100 by manipulating the actuation lever 402.

In particular, the tapering section 101 narrows in an imaginary dimension or plane, perpendicular to axis A.

The conduit 440 features - as depicted in figure 10c - a bend 403 along its longitudinal path 411 at an intermediate section between the mouthpiece opening 401 and the at least one pocket airflow exit 424a-b. This bend 403 reorients the longitudinal path 411 of the conduit 440, the longitudinal path 411 directed towards the mouthpiece opening 401, away from the side containing the opening of the at least one pocket airflow exit 424a-b.

The bend 403 modifies the direction of the conduit 440 by an angle ranging from 120 to 178 degrees, more precisely between 160 and 175 degrees, and even more specifically between 170 and 175 degrees, relative to the conduit's longitudinal path before and after the bend 403.

The pocket-side opening of the at least one pocket airflow exit 424a-b is positioned at an angle of approximately 90 degrees relative to the longitudinal path 411 of a straight section of the conduit 440 preceding the bend 403.

The mouthpiece 404 is in fluid communication with the collector window 425a-b, enabling the patient to inhale a medicament-air mixture. This mixture contains at least part of the medicament released from the opened pocket 202b#x, which is located at the collector window 425a-b, exemplified in figures 10a to 10c.

The following definitions outline the distinct dose types associated with the dry powder inhaler and their interrelationships, providing a clear framework for understanding the progression from the initial medicament load to the final therapeutic delivery.

Nominal Dose: The nominal dose is defined as the total quantity of the active pharmaceutical ingredient (API) or medicament formulation contained within a single pocket or blister on the dose strip prior to activation of the inhaler device or apparatus 100. This represents the full pre-measured quantity of the drug substance, for example in powder form, intended for delivery. The nominal dose serves as the starting point for all subsequent dose definitions, as it constitutes the total available quantity of the medicament in the device before any emission or inhalation takes place.

Emitted Dose: The emitted dose is defined as the portion of the nominal dose that is released from the apparatus 100 upon activation and is expelled through the device's mouthpiece in the form of a medicament-air mixture. Not all of the nominal dose becomes the emitted dose due to retention of powder in the pocket, device pathways, or other components of the delivery mechanism. The emitted dose is a subset of the nominal dose: Emitted Dose = Nominal Dose - Retention in pocket and/or device. The emitted dose represents the quantity available to be inhaled by the patient.

Inhaled Dose: The inhaled dose is defined as the fraction of the emitted dose that is successfully entrained in the airflow during inhalation by the patient and delivered through the device's mouthpiece. This dose reaches the patient's oropharyngeal cavity as a part of the medicament-air mixture. The inhaled dose is a subset of the emitted dose: Inhaled Dose = Emitted Dose - Loss at the Mouthpiece or Due to Inhalation Dynamics. The inhaled dose reflects the portion of the emitted dose that is accessible to the patient during use.

Fine Particle Dose (FPD): The fine particle dose (FPD) is defined as the portion of the inhaled dose that consists of particles small enough (typically < 5 µm aerodynamic diameter) to reach the deep lung regions for pulmonary deposition. These particles are critical for achieving therapeutic effects, as larger particles are likely to deposit in the upper airways and contribute less to the intended pharmacological action. The fine particle dose is a fraction of the inhaled dose: FPD = Inhaled Dose x Fraction of Particles < 5 µm. The FPD determines the efficacy of pulmonary drug delivery, as it quantifies the amount of drug reaching the target regions in the lungs.

Summary of Dose Hierarchy and Relationships: Nominal Dose: The starting point, representing the total pre-measured amount of medicament in a single blister. Emitted Dose: A fraction of the nominal dose, representing the medicament released from the device. Inhaled Dose: A subset of the emitted dose, representing the medicament that reaches the patient's oropharynx in the air stream. Fine Particle Dose: A fraction of the inhaled dose, representing the respirable particles that deposit in the deep lungs.

Example Equation for Dose Relationships: FPD = (Nominal Dose - Retention in Device) x Inhalation Efficiency x Fine Particle Fraction.

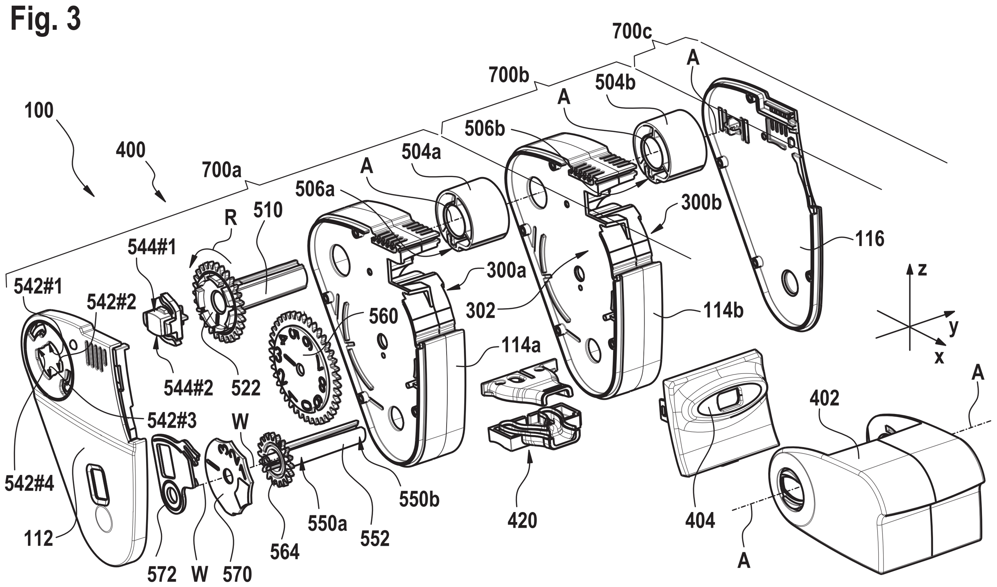

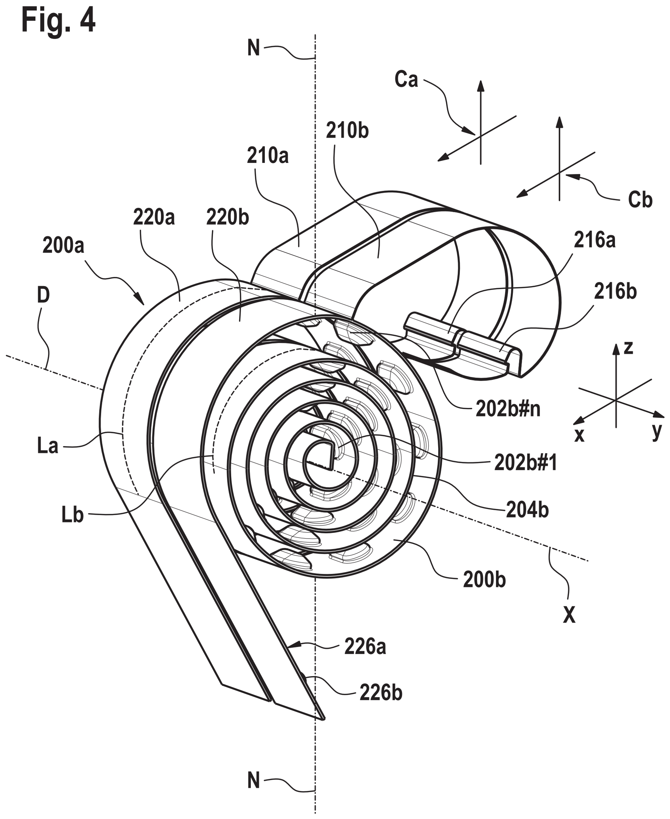

Figure 3 depicts the apparatus 100 in an exploded view. Figure 4 depicts the medicament carriers 200a-b in a perspective view in their start positions. The start position is the position of the strips or medicament carriers 200a-b in the factory before inserting into the apparatus 100. The medicament carriers 200a-b are arranged as if they are located in the apparatus 100. In the following reference is made to figure 3 and 4.

Each medicament carrier 200a-b comprises a plurality of closed pockets 202a#1-n, 202b#1-n along an imaginary curved line La-b of the medicament carrier 200a-b, wherein the closed pockets 202a#1-n, 202b#1-n contain the medicament in powder form. Each of the medicament carriers 200a-b are made from a thin lid foil 210a-b and a thicker base foil 220a-b. The pockets 202 formed into the base foil 220a-b contain the drug powder and are closed by the two foils, the lid foil and the base foil, locally attached to each other, for example welded together.

For the sake of clarity, the composition of the reference signs is exemplified in the following based on the reference signs 202a#1-n and 202b#1-n for the medicament carrier. In the example in Figure 4, the indices a and b each indicate one of the two strips or medicament carriers. The index 1-n means that there is a plurality of 1 to n pockets. The number of closed pockets is reduced during use of the apparatus 100, i.e. open pockets are created. Nevertheless, reference is made to the same pocket via the reference sign. Whether the pocket is opened or closed is indicated in the respective context. Moreover, even if individual pockets, e.g. with the reference sign 202a#1, are not shown in Figure 4, reference is nevertheless made to them in this description.

Inside the apparatus 100 at least one sealed pocket chamber 300a-b is configured to accommodate free-ended coiled-up portions 204a-b of the plurality of elongated medicament carriers 200a-b, wherein imaginary centre planes Ca-b spanned by the respective curved line La-b of the associated free-ended coiled-up portions 204a-b are spaced apart at least in the area of the at least one sealed pocket chamber 300a-b. The respective at least one free-ended coiled-up portion 204a-b comprises at least partly the closed pockets 202a#1-n, 202b#1-n. The curved line La-b extends along the closed pockets 202a#1-n, 202b#1-n of the respective medicament carrier 200a-b.

The elongated medicament carriers 200a-b are stacked on each other along a stacking axis X that runs perpendicular to the imaginary centre planes Ca-b.

In the case of several sealed pocket chambers 300a-b these chambers are stacked on each other along the stacking axis X.

The free-ended coiled-up portion 204a-b comprises the lid foil 210a-b adhered to the base foil 210a-b, thereby closing the pockets 202a-b. The medicament carrier 200a-b comprises the free-ended coiled-up portion 204a-b, the free-ended peeled-off lid foil or free-ended lid foil portion 210a-b, and the free-ended base foil or free-ended base foil portion 220a-b with opened pockets 202a-b.

In particular, separate neighbouring sealed pocket chambers 300a-b are provided for accommodating a respective one of the free-ended coiled-up portions 204a-b. The neighbouring sealed pocket chambers 300a-b are separated by a separation wall 302, wherein the separated sealed pocket chambers 300a-b are configured to accommodate at least the free-ended coiled-up portion 204a-b of the respective one of the plurality of elongated medicament carriers 200a-b.

A dispensing mechanism 400 is configured to act on the plurality of elongated medicament carriers 200a-b upon an actuation of the manual actuation lever 402 to dispense at least one of the medicament doses carried by the plurality of elongated medicament carriers 200a-b.

The dispensing mechanism 400 comprises a shared transport mechanism 500 that is configured to pull simultaneously a plurality of peeled-off lid foils 210a-b causing a simultaneous transport of at least one opened pocket 202a-b of an associated base foil 220a-b of the plurality of medicament carriers 200a-b.

At least one peeling edge 410 is configured, together with at least one transport mechanism 500, to peel off a lid foil 210a-b of the at least one medicament carrier 200a-b from a base foil 220a-b of the at least one medicament carrier 200a-b thereby uncovering at least one opening of at least one pocket 202 in the base foil 220a-b, wherein the medicament is accommodated in the at least one opened pocket 202. For example, the peeling edge 410 includes a rounded edge over which the lid foil 210a-b passes. Since the base foil 220a-b is configured to be more rigid than the lid foil 210a-b, the lid foil 210a-b is detached from the base foil 220a-b and the more rigid base foil 220a-b is transported onwards.

The coiled-up portion 204a-b comprises a distal end 244a-b of the whole medicament carrier 200a-b. A leading end of the lid foil 210a is affixed to a transporter hub 504a.

Therefore, the at least one transport mechanism 500 is configured to pull the peeled-off lid foil 210a causing a transport of the at least one opened pocket 202 of the base foil 220a-b to a collector channel 340, 340a-b. Pulling the peeled-off lid foil also causes the uncovering of the at least one opening of the at least one pocket. The base foil 220a-b together with the at least one opened pocket is transported into the collector channel 340a-b. The collector channel 340a-b has an inner contour that is configured to let the base foil 220a-b together with its opened pockets 202 pass in its direction of travel. The collector channel 340a-b is arranged between the at least one sealed pocket chamber 300a-b and a collector 420.

The collector 420 delimits at least partly the collector channel 340a-b by closing an opening 346a-b of the collector channel 340a-b. The collector 420 is configured to collect the medicament from the open pocket 202 via an airflow that passes through the pocket 202 and configured to deliver the resulting medicament-air mixture towards a mouthpiece 404.

The mouthpiece 404 is in fluid connection with the collector 420, wherein the mouthpiece 404 is configured to allow the patient to inhale the medicament-air mixture provided via an airflow through the collector 420 and the pockets 202.

A further channel 342a-b connects the collector channel 340a-b with a wind-up chamber 344a-b for accommodating waste base foil 220a.

The at least one sealed pocket chamber 300a-b for accommodating a free-ended coiled-up part of the medicament carrier 200a-b is separated from the collector channel 340a-b by a chamber separation wall 304a-b. The distal end of the chamber separation wall 304a-b extends into the at least one associated sealed pocket chamber 300a-b and provides towards its end a slight additional bent 306a towards the centre of the sealed pocket chamber 300a-b.

The at least one sealed pocket chamber 300a-b follows at least partly a cylindrical form, wherein the at least one collector channel 340a-b follows a circular ring shape in a cross section perpendicular to the actuation axis A. Said circular ring shape of the at least one collector channel 340a-b is bent into the same direction as at least one wall 304a-b arranged between the at least one collector channel 340a-b and the at least one sealed pocket chamber 300a-b.

A feeder channel 350a-b is arranged between the sealed pocket chamber 300a-b and the collector channel 340a-b. An inlet of the feeder channel 350a-b delimits the respective sealed pocket chamber 300a-b for accommodating the medicament carrier 200a-b. The outlet of the feeder channel 350a-b leading to the collector channel 340a-b is defined at least by the tip of at least one peeling edge 410a-b.

The at least one transport mechanism 500 comprises the transporter hub 504a-b with an attachment portion 506a-b for securing a leading portion 216a-b of the lid foil 210a-b, wherein the transporter hub 504a-b is rotatable about the actuation axis A. Inside the sealed pocket chamber 300a-b, the medicament carrier 200a-b is constituted by the base foil 220a-b with medicament-filled pockets, and the lid foil 210a-b closing the medicament-filled pockets. Upon rotating the transporter hub 504a-b in a rotational actuation direction R, the lid foil 210a-b is drawn towards the transporter hub 504a-b causing the base foil 220a-b with the lid foil 210a-b adhered to it to move from the sealed pocket chamber 300a-b through the feeder channel 350a-b to the peeling edge 410a-b. After peeling-off the lid-foil 210a-b from the base-foil 220a-b, the base foil 220a-b with the opened pockets moves into the collector channel 340a-b.

A diameter of the at least one transporter hub 504a-b and a pitch between neighbouring pockets 202 of the at least one medicament carrier 200a-b are adapted to each other in such a way that by traversing an actuation angle range on the actuation axis A in the actuation direction causes the at least one of the plurality of pockets 202 of the base foil 220a-b to be opened and causes the at least one opened pocket 202 to arrive at the collector 420.

The medicament carrier 200a-b is advanced by means of the lid foil 210a-b being wound onto the transport hub 504a-b, which is turned a set number of degrees with each operation / actuation. The diameter of the transport hub 504a-b increases by twice the lid foil thickness with each complete turn it makes. Therefore, the pitch of the pockets is correspondingly increased to match each complete turn. The pitch between neighbouring pockets 202 of the at least one medicament carrier 200a-b increases towards the trailing end of the medicament carrier 200a-b.

As the wider tolerances with respect to the alignment of the pockets to the collector are accepted, these increased wider do not interfere with the protection of the next dose sealing. In particular, the distance between neighbouring pockets is adapted such, that the peel point for the next pocket is not reached when previous open pocket is arranged at the collector. Accordingly, the adaption of the distance advantageously protects the next dose from opening.

Moreover, the increased pitch towards the end of the base foil gives a longer seal towards end of life. This is a benefit in terms of improved moisture protection.

The at least one transport mechanism 500 comprises a plurality of transport hubs 504a-b, each with an attachment portion 506a-b configured to secure a leading portion 216a-b of the lid foil 216a-b, wherein the transport hubs 504a-b are rotatable about the actuation axis A.

The lid foil 210a-b is wound onto the associated one of the transport hubs 504a-b. The transport hubs 504a-b are turned a set amount when the manual actuation lever 402 in form of the mouthpiece cover is opened.

An initial rotational position of the attachment portion 506a-b of the respective transport hub 504a-b and a length of the respective lid foil 210a-b between the leading portion 216a-b and at least a part of the plurality of pockets 202a-b are matched such that upon the actuation of the manual actuation lever 402 the respective next one of the pockets 202a-b arrives at the collector 420 in order to dispense the medicament inside the respective pocket 202a-b.

Advantageously, the provided match of the initial rotational position of the transport hub and the distance between pockets and the leading portion of the lid foil provide the initial pre-set for the correct alignment of each of the pockets with respect to the collector.

The initial rotational position of the attachment portion 506a-b is provided by a form-fit between an outward clip part 508a-b that engages in its open position with a raised contour 109a-b of the component 114a-b, in particular of the chassis part. After inserting the end of the lid foil into an inward clip part 509a-b, the outward clip part 508a-b is pressed inwardly; releasing itself from the raised contour 109a-b and fixing the end of the lid foil 210a-b to the transporter hub 504a-b. Of course, the initial rotational position of the attachment portion 506a-b can be arrived at by different means during manufacturing, for example, by using a holding mechanism to temporarily fix the hub 504a-b in order to affix the attachment portion 506a-b to the hub 504a-b.

The at least one transport mechanism 500 comprises a shared transport shaft 510 to which the plurality of transport hubs 504a-b are attached.

The at least one dispensing mechanism 400 comprises the manual actuation lever 402 that is configured to provide a torque to the transport mechanism 400, especially configured to provide the manual torque to the transport shaft 510.

The manual actuation lever 402 is a movable cover configured to be rotatable about the actuation axis A and configured to cover a mouthpiece 404 in a first state and to uncover the mouthpiece 404 in a second state.

The dispensing mechanism 400 comprises the at least one peeling edge 410a-b configured to peel off the lid foil 210a-b of a respective one of the plurality of medicament carriers 200a-b from the base foil 220a-b of the respective one of the plurality of medicament carriers 200a-b thereby uncovering an opening of at least one pocket 202a#1-n, 202b#1-n in the respective base foil 220a-b, wherein the medicament is accommodated in the respective opened pocket 202a#1-n, 202b#1-n.

The parts of the apparatus 100, especially part of the dispensing mechanism 400, in particular the transport shaft 510, comprise material with Young's Modulus, is between 0.8 GPa and 2.0 GPa, especially between 1.0 GPa and 1.8 GPa, especially between 1.0 GPa and 1.5 GPa. Advantageously, the selected ranges for Young's Modulus allow the use of Mono-material Polyolefins or even plant-based polymers like Polyactic Acid, PLA, Polypropylene, PP, High-Density Polyethylene, HDPE, or another Polyolefin. Of course, these materials could be blended with one another or blended with additives like glass fibres.

Departing from the plant-based approach, some or all components of the apparatus 100 could be made from non-plant based polymers.

Some or all components of the apparatus 100 can be made of the same polymer for recycling purposes.

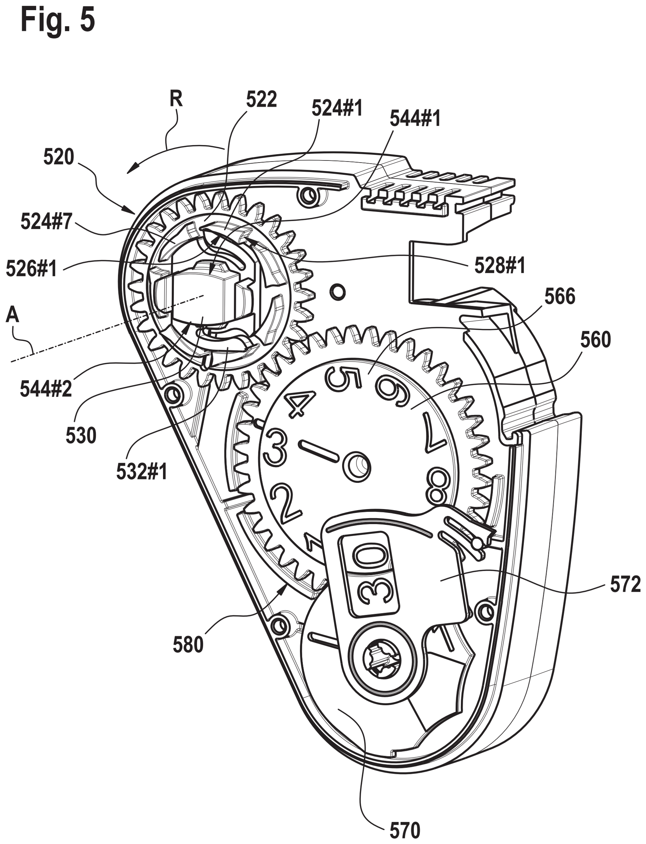

Figure 5 depicts a part of the apparatus 100 in a perspective view; in particular, a foil rewind prevention mechanism 520 is shown.

The at least one transport mechanism 500 comprises a foil rewind prevention mechanism 520 that is arranged between the manual actuation lever 402 and the remaining part of the transport mechanism 400, especially between the manual actuation lever 402 and the transport shaft 510. The foil rewind prevention mechanism 520 is configured to transfer the torque in an actuation direction R of rotation to the plurality of transport hubs 504a-b and configured to prevent torque from being transferred to the transport hubs 504a-b in the opposite direction of the actuation direction R of rotation.

According to the shown example, the foil rewind prevention mechanism 520 comprises a ratchet wheel 522 with a plurality of teeth 524#1-7, each one with a sliding portion 526#1-7 and an engagement portion 528#1-7, the plurality of teeth arranged along an imaginary circle around the actuation axis A, and a pawl 530 with at least one arm 532#1-2 rotatable about the actuation axis A, the at least one flexible arm 532#1-2 configured to pass the sliding portion 526#1-7 and configured to engage with the engagement portions 528#1-7 in order to transfer the torque to the transport shaft 510.

An actuator rotation limiting mechanism 540 comprising first limiting means 542#1-4 is configured to delimit a rotational movement of the manual actuation lever 402 to a first angular range. The first limiting means 542#1-4 are delimiting surfaces of a through-hole of a drive cover 112 and interact with the counter surfaces 544#1-2 of the pawl 530. The plurality of teeth 524#1-7 configured to delimit a rotational movement of the transport mechanism 400, especially of the shaft 510, to a second angular range. The teeth 524#1-7 define the second angular ranges by the length between neighbouring engagement portions 528#1-7.

A ones gear 560, especially a single units gear, meshes with a drive gear or transporter gear 522 that is rigidly connected to the transport shaft 510. The ones gear 560 further meshes with a base winder gear 564 that is rigidly connected to the base winder shaft 552. The ones gear 560 and the base winder gear 564 comprise a human readable numbering representing the numbers of doses that have been already consumed with the apparatus 100 or are still available. Upon turning hub 504a-b by 1/7^{th}, the ones gear 560 below is moved 1/10^{th}.

A decimal disc or tens disc 570 is connected to the base winder gear 564. The readable numbering on the decimal disc represents the decimal steps of used doses or still available doses. Furthermore, a flag 572 covers at least partly the decimal disc or tens disc 570 and the ones gear 560. Towards the end of the available doses, in particular after the last dose, the flag 572 is engaged by corresponding contours of the flag 572 and the decimal disc or tens disc 570. Therefore, after the last dose, the flag 572 is moved to the visible area. When the red flag 572 has moved to the visible area, the now visible red flag 572 indicates to the patient that he has to get a new device.

The apparatus 100 features the dose counter that provides a progressive warning to the user as the device approaches the end of its available doses. The dose counter includes the ones gear 560 and the tens disc 570 that interact to display the remaining doses. When the remaining doses drop below 10, a red-colored section of the tens disc 570 becomes visible, providing an early visual warning to the user. Upon depletion of the last dose, the red flag 572 is triggered and moves into the visible area, covering the tens disc 570 and ones gear 560 to indicate that no doses remain.

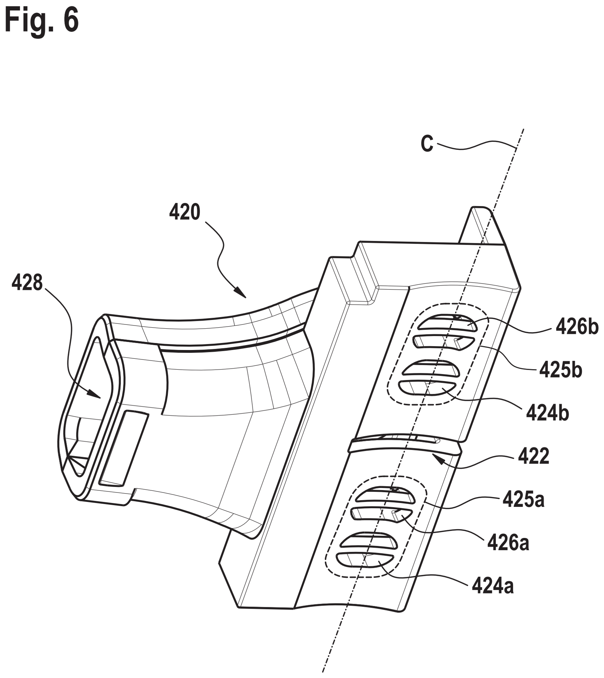

This dose counter ensures the user is gradually and clearly informed of the device's status, facilitating timely replacement and uninterrupted treatment. The mechanism is directly linked to the transport system for accurate dose tracking and utilizes a simple and reliable engagement system to activate the warning indicators. This design enhances user safety, usability, and continuity of treatment. Figure 6 depicts the shared collector 420. The shared collector 420 is configured to collect the medicament from an opened pocket 202 of the each base foil 220a-b via an airflow that passes through the respective pocket 202 and configured to deliver the resulting medicament-air mixture towards the mouthpiece 404.

The collector 420 comprises at least one port section 422 interrupted at least by a plurality of pairs of at least one inlet port or pocket airflow entry 426a-b and at least one outlet port or pocket airflow exit 424a-b, wherein the pairs are arranged such that, in use, underpressure on the side of the corresponding outlet port or pocket airflow entry 424a-b causes air to be sucked through the inlet port or pocket airflow entry 426a-b, the air passing through the open pocket 202 thereby creating a medicament-air-mixture, and the medicament-air-mixture exiting through the outlet port 424a-b towards the mouthpiece 404. The outlet ports 424a-b are in fluid connection with a collector outlet 428 that leads to the opening of the mouthpiece 404.

The terms "inlet" and "outlet" with regard to the ports 424a-b and 426a-b are to be understood from the perspective of the respective pocket. Seen from the mouthpiece, the ports 434a-b and 424a-b are inlet ports.

The plurality of pairs, especially the respective inlet and outlet ports, are arranged along a collector axis C that is parallel to the actuation axis A.

The at least one port section 422 comprises at least one concave surface especially extending parallel to the collector axis C. The concave surface delimits the collector channel 340a-b and enables that the bent base foil 220a-b may contact the concave surface due to its convex shape seen from the opening of the pocket in the base foil 220a-b.

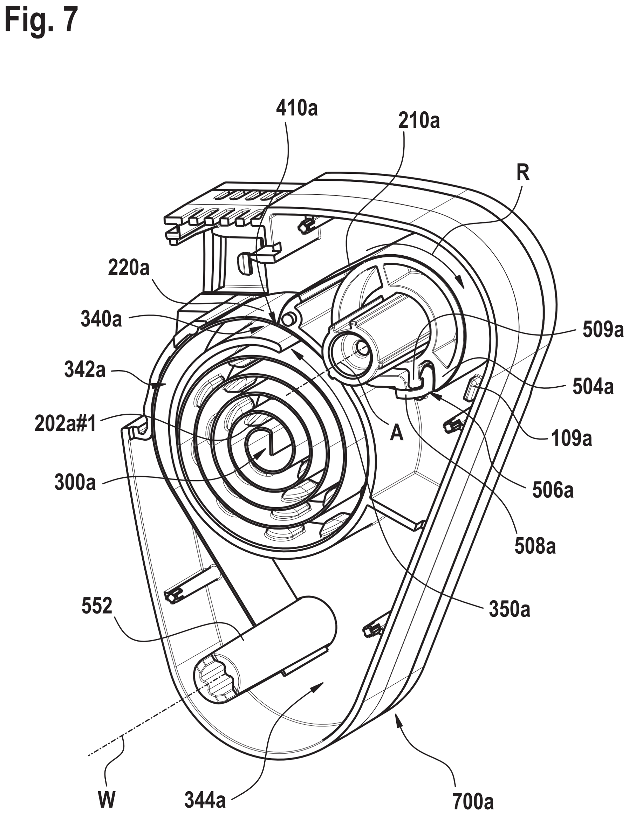

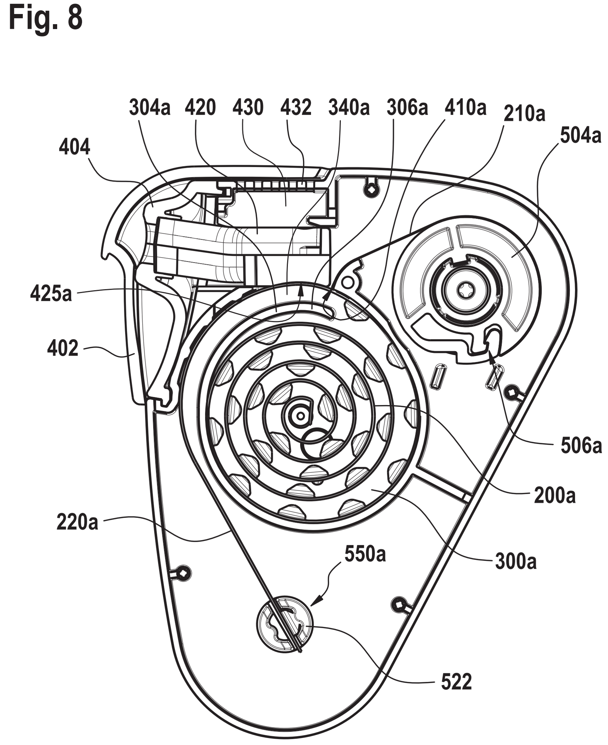

Figure 7 depicts the inner construction of the apparatus 100 in a perspective view. Figure 8 depicts the apparatus 100 in a sectional view. In the following, the description refers to figures 7 and 8.

The transport mechanism 500 comprises a plurality of base winder sections 550a-b, each base winder section 550a-b comprising an attachment portion for securing a leading portion 226a-b of an associated base foil 220a-b of the plurality of medicament carriers 200a-b, wherein the base winder sections 550a-b are rotatable about a wind-up axis W.

A spare pocket of the base foil 220a-b locates into the associated base winder section to provide engagement. The waste part of the base foil is loosely wound by the base winder sections 550a-b. A shared wind-up shaft 552 provides the plurality of wind-up sections 550a-b. The base winder sections 550a-b and the transport shaft 510 are motion-mechanically connected with each other.

The apparatus 100 comprises: the at least one elongated medicament carrier 200a-b, wherein the at least one medicament carrier 200a-b comprises the plurality of closed pockets 202a#1-n, 202b#1-n that extend along the at least one medicament carrier 200a-b, wherein the closed pockets 202a#1-n, 202b#1-n contain a medicament in powder form, wherein the closed pockets 202a#1-n, 202b#1-n are formed by the recesses in the base foil 220a-b, and wherein the lid foil 216a-b closes the plurality of openings of the recesses in the base foil 220a-b. There is provided the peeling edge 410 configured, together with the manual actuation lever and the transporter hub 504a-b, to peel off the lid foil 210a-b of the at least one medicament carrier 200a-b from the base foil 220a-b thereby opening one of the plurality of closed pockets 202 in the base foil 220a-b. There is provided the transporter hub 504a-b that is operated by the manual actuation lever, wherein the transporter hub 504a-b comprises the attachment portion 506a-b for securing a leading portion 216a-b of the lid foil 210a-b, and wherein the transporter hub 504a-b is configured, together with the peeling edge 410, to move the opened pocket 202a#1-n, 202b#1-n of the base foil 220a-b toward a collector window 425a-b solely via a tensile force coupled into the lid foil 210a-b by means of the transporter hub 504a-b. The foil rewind prevention mechanism 520 is configured to prevent an unintended rewind of the lid foil 210a-b. The apparatus 100 comprises the at least one collector window 425a-b, over which the respective opened pocket 202a#1-n, 202b#1-n is moved. There is provided the mouthpiece 404 in fluid connection with the collector window 425a-b, wherein the mouthpiece 404 is configured to allow the patient to inhale a medicament-air mixture comprising powder from the at least one opened pocket 202a#1-n, 202b#1-n arranged at the collector window 425a-b.

The tensile force coupled into the lid foil 210a-b is transmitted into the portion of the medicament carrier 200a-b where the base foil 220a-b and the lid foil 210a-b are still adhered to each other. That is why the base foil 220a-b with its opened pocket is moved toward the collector window 425a-b.

A distance between the peeling edge 410 and the associated collector window 425a-b is smaller than a distance between two neighbouring pockets 202a#1-n, 202b#1-n.

An attachment position of the transporter hub 504a-b for the leading portion 216a-b and a distance along the medicament carrier 200a-b between the leading portion 216a-b of the lid foil 210a-b and the next pocket along the medicament carrier 200a-b are configured such that the next pocket is aligned with the collector window 425a-b within at least one or more actuations of the manual actuation lever.

The peeling edges 410a-b have a soft radius between 0.15 mm and 0.35, in particular between 0.19 mm and 0.31 mm, in particular between 0.20 mm and 0.30 mm. A pitch between neighbouring pockets increased towards the end of the medicament carrier 200a-b. This pitch pattern guarantees that on each 1/7^{th} turn of the transporter hub 504a-b, one pocket is opened and transported to the associated collector window 425a-b.

Each turn of transporter hub 504a-b, the thickness of the transporter hub 504a-b increases. By increasing the pitch there is a compensation in order to keep the pockets aligned to the associated collector window 425a-b.

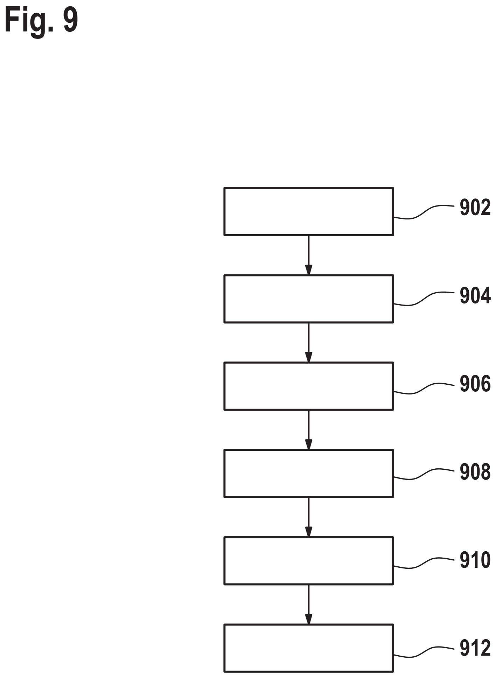

Figure 9 depicts a schematic flow diagram for manufacturing the apparatus 100 of the previous figures. As can be seen in figure 1, a component 114a of a first sub-assembly 700a provides the first sealed pocket chamber 300 and a second component 114b of a second sub-assembly 700b provides the second sealed pocket chamber 300b. The components 114a-b are identical in their form, thereby reducing costs associated with manufacturing. The main body 110 comprises at least the drive cover 112, the first component 114a, the second component 114b, and a strip cover 116.

Mounting 902 of the first sub-assembly 700a, that comprises a first one of the plurality of sealed pocket chambers 300a, involves an assembly of the drive components with the first component 114a. For example, the drive or transport shaft 510 and the wind-up shaft 552 are passed through the assigned openings of the first component 114a. Moreover, the ones gear 560 is mounted in order to connect and mesh with the drive gear or transporter gear 522 and the base winder gear 564. Additionally, the drive cover 112 closes the first sub-assembly 700a on one side. The transporter hub 504a is mounted on the drive or transport shaft 510.

Inserting 904 the first medicament carrier 200a into the first one of the plurality of sealed pocket chambers 300a comprises inserting the free-ended coiled-up portion of the medicament carrier 200a into the sealed pocket chamber 300a, fixing the leading end of the lid foil 210a to the transporter hub 504a, and fixing the leading end of the base foil 220a to the base winder 552. After both steps 902 and 904, the partly manufactured apparatus 100 comprising the first sub-assembly 700a and the first medicament carrier 200a is shown in figure 7.

Mounting 906 of the second sub-assembly 700b, which comprises a second one of the plurality of sealed pocket chambers 300b onto the first sub-assembly 700a, comprises Passing of the drive or transport shaft 510 and of the wind-up shaft 552 through assigned openings of the second component 114b, thereby closing the first sealed pocket chamber 300a. The transporter hub 504b is mounted on the drive or transport shaft 510.

Inserting 908 the second medicament carrier 200b into the second one of the plurality of sealed pocket chambers 300b comprises inserting the free-ended coiled-up portion of the medicament carrier 200b into the sealed pocket chamber 300b, fixing the leading end of the lid foil 210b to the transporter hub 504b, and fixing the leading end of the base foil 220b to the base winder 552.

After inserting 908, a third sub-assembly 700c, in particular a strip cover 116, is mounted 910 onto the second sub-assembly 700b.

In step 912, the collector 420 and the mouthpiece 404 are inserted into the pre-assembled apparatus 100. Moreover, the manual actuation lever 402 is rotatable connected to the apparatus 100.

The inserting 904, 908 of the respective medicament carrier 200a-b comprises: Temporarily fixing a transport hub 504a-b in a pre-defined position with respect to the associated sub-assembly 700a-b; and Attaching a leading portion 216a-b of the lid foil of the respective medicament carrier 200a-b to an attachment portion 506a-b of the temporarily fixed transport hub 504a-b. Advantageously, the manufacturing method involves the fixing of the transport hub in order to precisely attach the lid foil, which is responsible for the transport of the pockets, to the transport hub. This precise attachment method of the lid foil defines the individual positions of the pockets with respect to the collector.

Figures 10a-c depict different alignment positions of pockets of the base foil with respect to the collector 420. For example, figure 10a depicts an open pocket 202b#x of the base foil 200b that is aligned with the collector inlet 426b. According to an example, the alignment involves that the inner walls of the collector inlet 426b are flush with the inner surface of the pocket 202b#x at the opening of the pocket 202b#x. In contrast, figure 10b depicts a retarded pocket 202b#x and figure 10c depicts an advanced pocket 202b#x. Both the retarded and advanced pockets are depicted such that more or less the half of the opening of the pocket 202b#x overlaps with the collector inlet 426b and the corresponding collector outlet (not shown). The alignment situations of the pocket 202b#x with respect to the collector 420 and its outlets and inlets are accepted as the drug dose delivery is guaranteed and variations in dose delivery are reduced. A retarded or advanced pocket has no negative effect on dose delivery.

The apparatus 100, as depicted in figure 10a, includes at least one elongated medicament carrier 200a-b. This medicament carrier 200a-b comprises a plurality of closed pockets 202a#1-n, 202b#1-n, which extend along the length of the medicament carrier. Each closed pocket 202a#1-n, 202b#1-n is designed to contain a medicament in powder form.

The mouthpiece 404 features an outer opening 405 that connects to at least one conduit 440. This conduit 440 extends through the collector 420, beginning at the entry section 442 and terminating at the mouthpiece opening 405.

The at least one collector 420 is provided, incorporating the at least one conduit 440 which extends along a longitudinal axis LA through the collector 420 from at least one entry section 442 towards the opening 405 of the mouthpiece 404.

The entry section 442 of the conduit 440 is characterised by the at least one collector window 425a-b, over which the respective opened pocket 202a#1-n, 202b#1-n, exemplified in figures 10a-c, is positioned, the collector window 425a-b comprising at least one pocket airflow entry 426a-b and at least one pocket airflow exit 424a-b. The pocket airflow entry 426a-b is configured to facilitate the introduction of air into an opened pocket 202a#1-n, 202b#1-n of an elongated medicament carrier 200a-b arranged at the collector window 425a-b. The pocket airflow exit 424a-b, on the other hand, is designed to allow the medicament-air mixture to exit the opened pocket 202a#1-n, 202b#1-n and enter the conduit 440.

Furthermore, the pocket airflow exit 424a-b features at least one pocket-side opening, where the imaginary normal axis N of the opening or its representative area is generally oriented orthogonally to the longitudinal axis LA of the conduit 440, with an allowable deviation of up to ±45°, more specifically ±35°, and particularly ±10°, from the orthogonal orientation. This precise angular alignment ensures optimal airflow and mixing efficiency as the medicament-air mixture is directed through the conduit.

A pair of parallel, imaginary outer planes 450, 452 extend through the outermost portions 454, 456 of the inner wall of the conduit 440, defining the spatial boundaries of the conduit 440. The entry section 442 of the conduit 440 includes the at least one collector window 425a-b, over which the respective opened pocket 202a#1-n, 202b#1-n is moved.

The collector window 425a-b contains at least one pocket airflow entry 426a-b and at least one pocket airflow exit 424a-b. The airflow entry 426a-b is designed to direct air into the opened pocket 202a#1-n, 202b#1-n of the elongated medicament carrier 200a-b, positioned at the collector window 425a-b.

Conversely, the pocket airflow exit 424a-b is configured to allow the medicament-air mixture from the opened pocket 202a#1-n, 202b#1-n to flow into the conduit 440. The airflow exit 424a-b is characterised by at least one pocket-side opening, whose imaginary normal axis N is orthogonal to both parallel imaginary outer planes 450, 452. The normal axis N determines the orientation of the pocket-side opening in relation to the conduit 440.

In figure 10a, the outermost portion 454 of the conduit's inner wall is adjacent to the mouthpiece opening and positioned on the side opposite the pocket airflow exit 424b. The outermost portion 456, on the other hand, is located near the airflow exit 424b.

In figure 10b, a pair of parallel, imaginary inner planes 460, 462 extend through the innermost portions 464, 466 of the inner wall of the conduit 440. In this case, the pocket airflow exit 424a-b also includes a pocket-side opening, with its imaginary normal axis N positioned orthogonally to both inner planes 460, 462.

In this embodiment, the innermost portion 464 of the inner wall is positioned opposite the airflow exit 424b, while the innermost portion 466 is adjacent to the mouthpiece opening, situated on the same side of the collector 420 as the airflow exit 424b.

The dry powder inhaler or apparatus 100 was evaluated for its performance based on the position of the pocket 202 containing the medication in powder form relative to the inlets and outlets of the collector. The findings are presented in the following table. The table includes columns for the best case scenario, labelled "aligned", which serves as the 100% reference point. Additionally, there are two more columns with measurements for two different positions of the pouch: one labelled "retarded" and another "advanced" according to the situations depicted in Figures 10b and 10c. These values are relative to the ideal alignment or position of the pocket.

**Table 1: Emitted Dose Parameters**

| | - aligned - | - retarded - | - advanced - |
|---|---|---|---|
| Total ex-device | 100,0% | 102,5% | 99,4% |
| ISM | 100,0% | 102,8% | 98,3% |
| Stages 3-MOC | 100,0% | 103,2% | 97,8 % |
| "% Recovery | 100,0% | 102,5% | 99,4% |

The results indicate good performance for the specified parameters in these positions. Each of the parameters shown in the rows of the table provides different insights into the inhaler's effectiveness and efficiency in delivering the medication from the device to the user under varying conditions of pocket alignment:

| | |
|---|---|
| "Total ex-device" | refers to the total amount of medication that exits the device, which is a crucial measure of the inhaler's efficiency. Here, the aligned case is the reference for the retarded and advanced cases; |
| "ISM" | refers to the Impactor-Sized Mass that is the sum of the mass of a particular API (active pharmaceutical ingredient) recovered from the Next Generation Pharmaceutical Impactor NGI; |
| "Stages 3-MOC" | refers to a fine particle fraction according to the Next Generation Pharmaceutical Impactor; |
| "% Recovery" | describes the percentage of the initial dose that is successfully delivered through the inhaler. |

The presence of these parameters demonstrates a thorough evaluation of the inhaler's performance in delivering medication under different conditions, which is vital for ensuring effective treatment for users.

Tests have demonstrated that the shown retarded or advanced pocket alignment, respecting accepted tolerances, do not change the emitted dose. Therefore, there is no need for small tolerances or complicated mechanism to achieve an exact alignment position of the pockets.

For example, the pre-bent medicament carriers 200a-b interact with the bent surface of the port section 422 of the collector 420 in order to provide at least a temporal contact between the bent surface of the collector and the surface of the base foil 200a-b surrounding the active opened pocket. This temporal contact is caused by the underpressure inside the collector when the patient sucks in the air in order to obtain the emitted dose.

The apparatus 100 further comprises an intermediate air chamber 430 delimited by an air inlet section 432 (shown in Figure 8) of the apparatus 100, the plurality of air inlet ports 426 a-b, and by-pass ports 434a-b. The remaining inner delimiting walls of the intermediate air chamber 430 are sealed with respect to the other components of the apparatus 100.

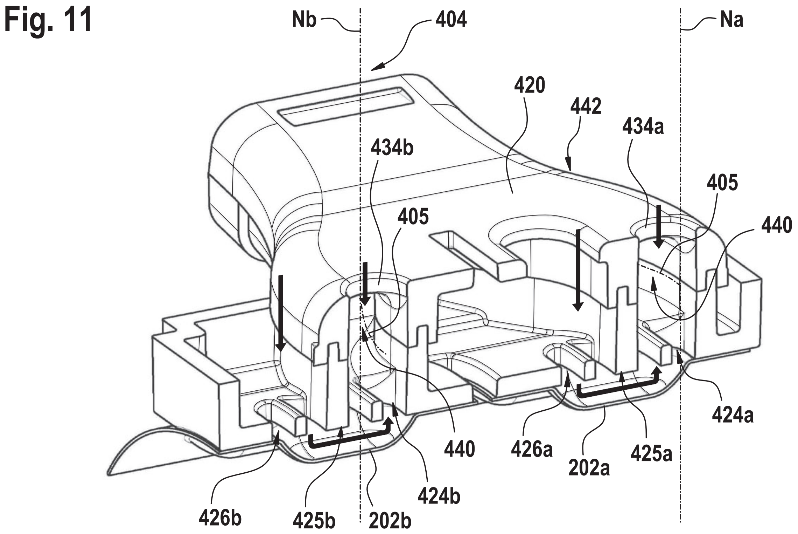

Figure 11 depicts an exemplary air flow through the pockets towards the mouthpiece.

Openings of the inlet and outlet ports 426a-b, 242a-b, in particular of a plurality of collector windows 425a-b, that face to the collector channel, follow the at least one concave surface.

Each inlet port 426a-b of a plurality of collector windows 425a-b is arranged towards one side of the apparatus 100, wherein each outlet port 424a-b of the plurality of collector windows 425a-b is arranged toward the other side of the apparatus 100.

In the shown example, the imaginary surfaces of the plurality of collector windows 425a-b coincide.

The collector 420 depicted in figure 11 comprises: at least one conduit 440 extending through the collector 420 from at least one an entry section 442 toward a mouthpiece 404; wherein the entry section 442 of the conduit 440 comprises: at least one collector window 425a-b with at least one pocket airflow entry 426a-b and at least one pocket airflow exit 424a-b, the pocket airflow entry 426a-b configured to receive air to enter an opened pocket 202a#1-n, 202b#1-n of an elongated medicament carrier 200a-b arranged at the at least one collector window 425a-b, the at least one pocket airflow exit 424a-b configured to receive a medicament-air mixture from the opened pocket 202a#1-n, 202b#1-n to enter the at least one conduit 440; and at least one bypass port 434a-b configured to receive air to enter the at least one conduit 440, wherein a distance D between the at least one bypass port 434a-b and the at least one pocket airflow exit 424a-b is at least greater than an equivalent diameter of an imaginary circular opening having the same cross-sectional area as the associated at least one pocket airflow exit 424a-b and/or wherein the at least one bypass port 434a-b and the at least one pocket airflow exit 424a-b lead into opposing sides of the at least one conduit 440.

When the manual actuation lever, here in form of the mouthpiece cover, is opened the transport hub and the transporter gear are rotated 51.43 degrees (one seventh of a full turn). This moves the strip or medicament carrier one pocket pitch.

The ones gear is an idler taking the drive to the base spool.

The ones gear is arranged to have the correct number of teeth such that it is turned one tenth of a turn with each operation. This enables it to show a count for each pocket.

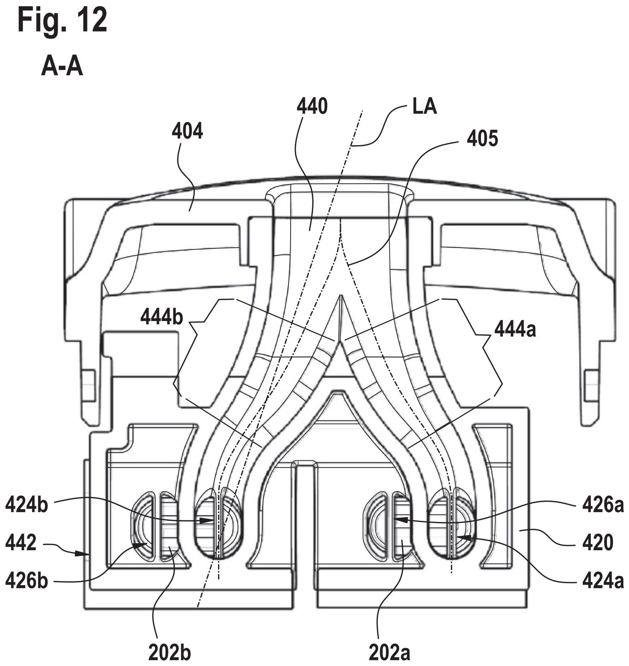

Figure 12 depicts a sectional drawing of the collector 420 and the mouthpiece 404, with the section along the conduit 440. The at least one conduit 440 comprises at least one diffusor section 444a-b along an air path from the entry section 442 towards the mouthpiece 404.

The diffusor section 444a-b comprises an increase in diameter of the conduit 440 along the direction towards the mouthpiece 404.

In the shown example, the conduit 440 is Y-shaped with two legs of the conduit 440 extending to individual pocket airflow exits 424a-b.

The diameter increase of the at least one diffusor section is implemented along an imaginary plane A-A arranged between the at least one bypass port 434a-b and at least one pocket airflow exit 424a-b.

The diameter increase of the at least one diffusor section 444a-b is mainly implemented by the collector 420 in an area between two imaginary parallel planes that enclose the imaginary plane A-A. The two imaginary planes are arranged inside an area that is delimited by further imaginary planes that run through the at least one bypass port 434a-b and the at least one pocket airflow exit 424a-b.

The conduit 440 comprises a curvature or bend along its length, which defines an angle of less than 180° and more than 110°, in particular less than 180° and more than 150°, measured in a direction facing away from the at least one open pocket 202a#1-n, 202b#1-n. This is advantageous for the placement of the mouthpiece 404 that can face lightly upwards.

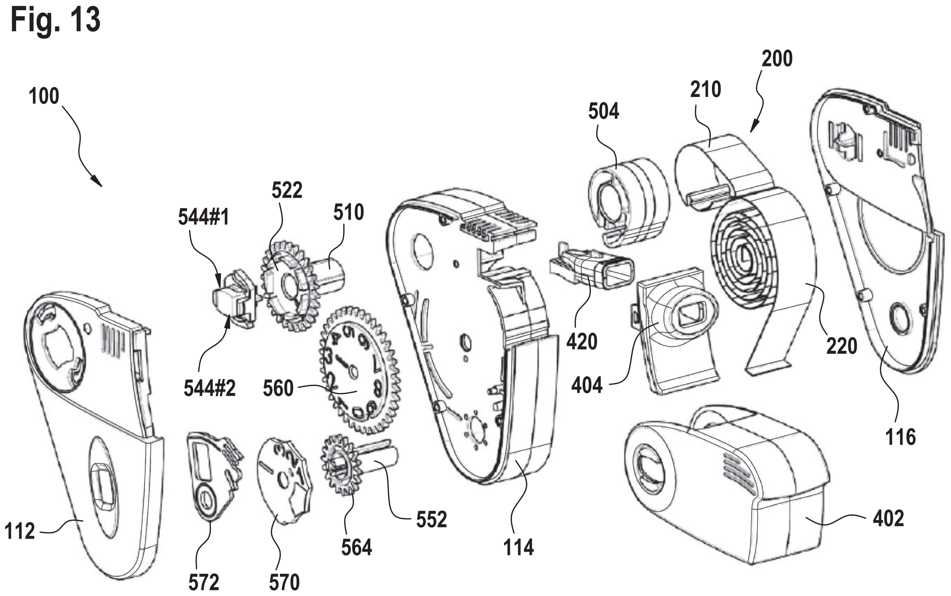

Figure 13 depicts, in an exploded view, an example of the apparatus 100 as a single-strip device. With reference to figure 3, the example of figure 13 comprises a reduced set of parts similar to those parts of the apparatus of figure 3. The lengths of the transport shaft 510 and of the base winder shaft 552 are shorter in comparison with the respective counterparts in figure 3. For the details of each part, reference is made to the above description.

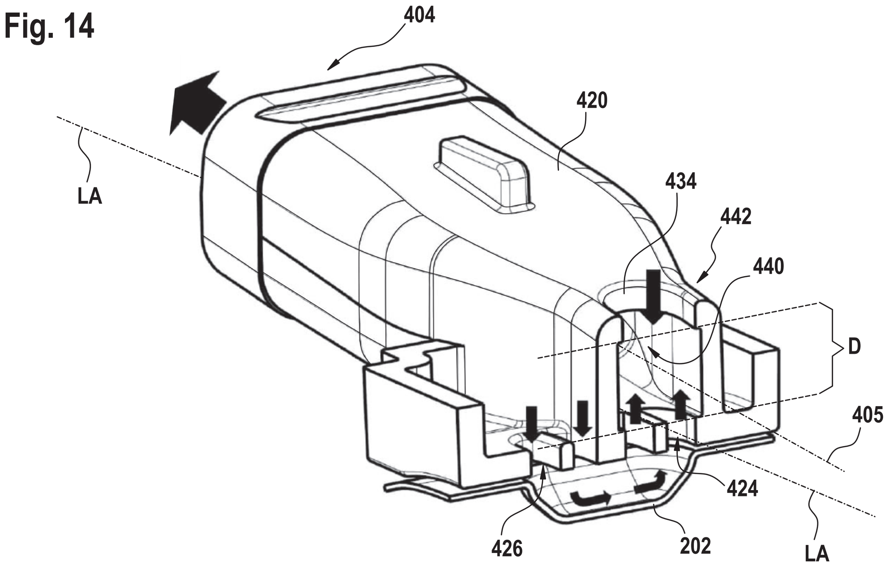

Figure 14 depicts the collector 420 of the apparatus 100 in figure 13. This collector 420 comprises the conduit 440 that comprises only a single fluid connection between the entry section and the distal end of the conduit 440 toward the mouthpiece 404. The features described in relation to the single-strip device and the collector 420 are also applicable to the multi-strip device and the associated collector 420 depicted in the figures preceding figure 13.

An open cross section of the at least one bypass port 434a-b is equal or greater than an open cross section of the associated at least one pocket airflow entry 426a-b or at least one pocket airflow exit 424a-b.

A distance D between the at least one bypass port 434a-b and the at least one pocket airflow exit 424a-b is equal or greater than 1.4 multiplied with the equivalent diameter of an imaginary circular opening having the same cross-sectional area as the associated at least one pocket airflow exit 424a-b.

The at least one bypass port 434a-b and the at least one pocket airflow exit 424a-b extend parallel to each other. In particular, imaginary planes through at least one opening of the ports 434a-b and 424a-b are parallel to each other with distance D.

Figure 5 and Figures 15 to 20 depict a dose counter 580 that indicates the remaining doses, or alternatively the used doses, to the user of the apparatus 100.

The dose counter 580 comprises: a manual actuation lever 402; a transporter gear 522 that is operated by the manual actuation lever 402, wherein the transporter gear 522 is configured to rotate a certain actuation angle for each actuation of a dose delivery of the at least one medicament, wherein each actuation opens at least one pocket 202a#1-n, 202b#1-n of an elongated medicament carrier 200a-b, wherein the at least one opened pocket 202a#1-n, 202b#1-n contains the at least one medicament and allows an airflow to expel the at least one medicament from the at least one opened pocket 202a#1-n, 202b#1-n; the ones gear 560 engaging with the transporter gear 522 and configured to rotate one tenth of a turn for each actuation of the transporter gear 522, wherein the ones gear 560 includes a disk-like section 566 that comprises human-readable numerals from 0 to 9 along an imaginary circular line; a tens disc 570 rotatably mounted, wherein the tens disc 570 is configured to rotate a certain angle upon each passage of the numeral 0 on the ones gear 560 to indicate the next decimal place of the count, wherein the ones gear 560 includes at least two numerals along an imaginary circular line, wherein the most neighbouring numerals displayed by the ones gear 560 and the tens disc 570 together represent the number of remaining or used doses of the at least one medicament; and an engagement mechanism between the ones gear 560 and the tens disc 570 configured to rotate the tens disc 570 upon or after the passage of the numeral 0 on the ones gear 560.

The ones gear 560 is configured to display ones or units of the decimal number of actuations remaining, and the tens disc 570 is configured to display the tens of the decimal number.

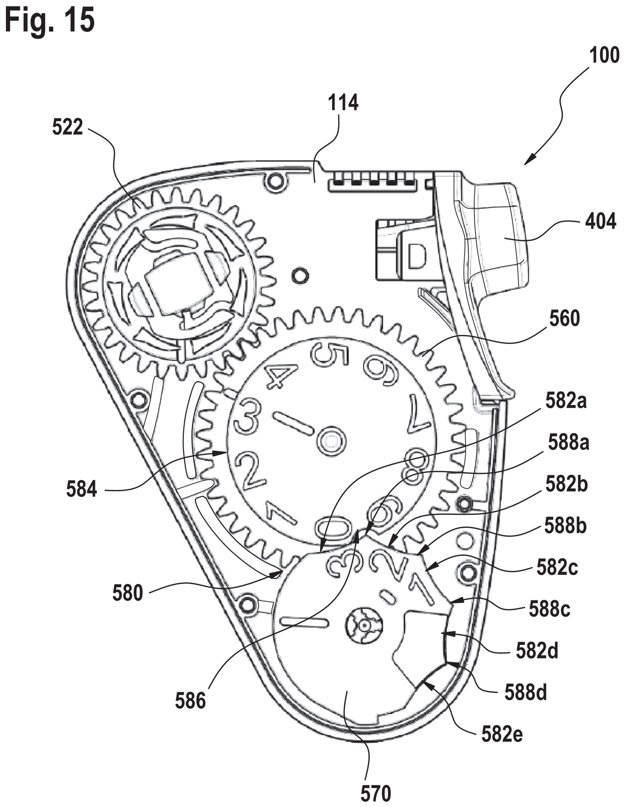

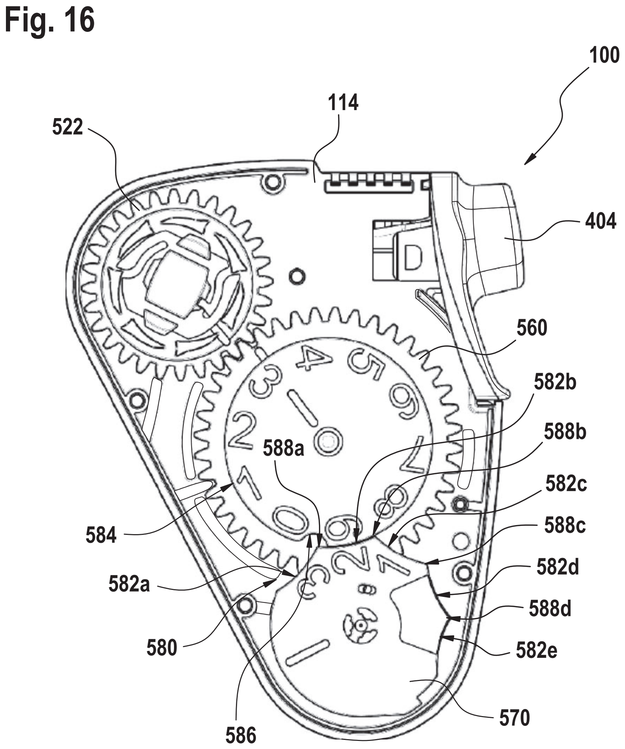

Figure 15 and 16 depicts the dose counter 580 in a transition from indicating the remaining doses of 30 to indicating the remaining doses of 29.

The tens disc 570 comprises a plurality of lateral recesses 582a-b following an outward contour 584 of the disk-like section 566 of the ones gear 560.

The tens disc 570 and the ones gear 560 are geared such that the tens disc 570 rotates by a predetermined angular increment, in particular one ninth of a turn, each time a section between numeral 0 and numeral 9 of the ones gear 560 passes.

An outward contour 584 of the ones gear 560 is interrupted by a recess 586, and wherein the recess 586 engages with a respective projection 588a-d of the tens disc 570, said projection 588a-d arranged between neighbouring recesses 582a-e of the tens disc 570.

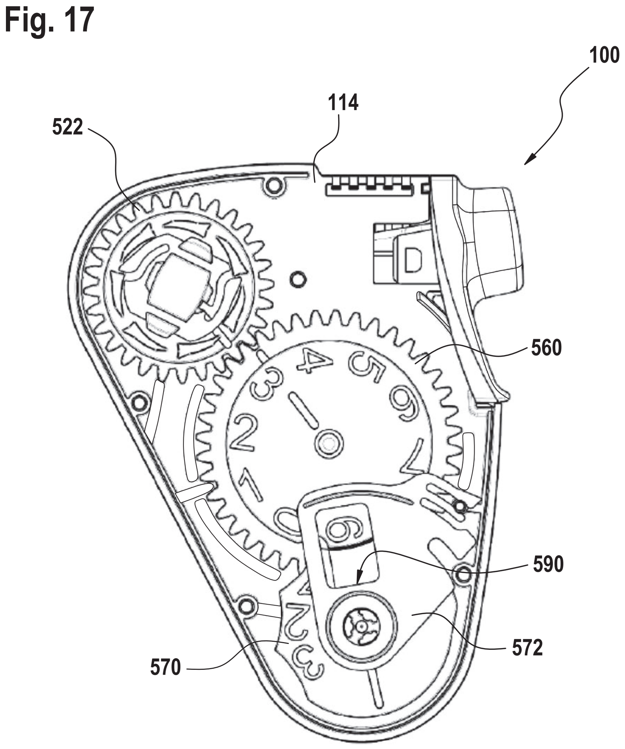

Figures 17 to 19 illustrate the function and mechanism of a rotatable flag 572 of the dose counter 580. The rotatable flag 572 rotates about the same axis as the tens disc 570 and covers the tens disc 570 at least partly.

A rotatable flag 572 is positioned to cover at least a portion of the ones gear 560 and the tens disc 570. An opening 590 in the rotatable flag is configured to display the most neighbouring numerals of the ones gear 560 and the tens disc 570.

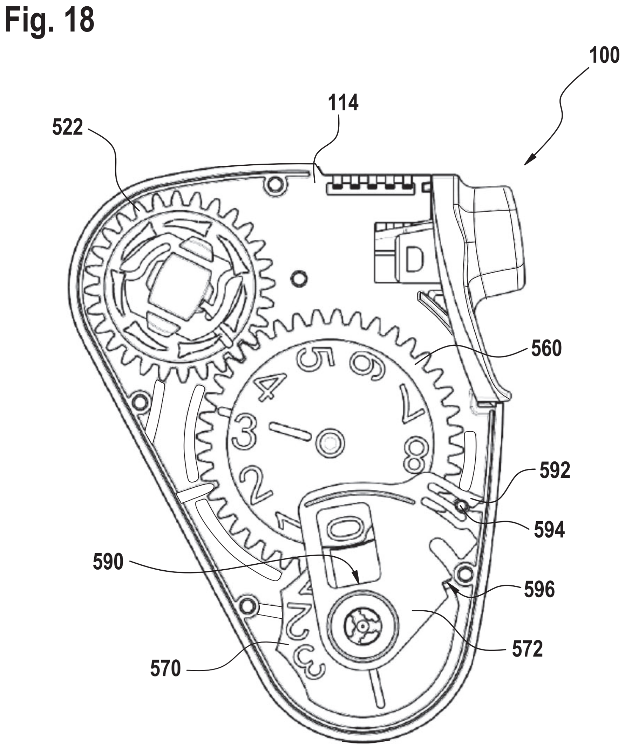

Figure 18 depicts that the rotatable flag 572 is secured in a primary position in which the opening 590 displaying both most neighbouring numerals of the ones gear 560 and the tens disc 570. The flag rotatable 572 is secured in the primary position by engaging with the housing. In the shown example, a clamp section 592 of the rotatable flag 572 is held in a force-fit and/or form-fit manner on a cylindrical section 594 of the housing.

The rotatable flag 572 is configured to rotate upon the engagement of a dog 596 of the rotatable flag 572 with the tens disc 570 after the last pocket is opened, such that the opening 590 in the flag 572 is rotated away to obscure the dose counter 580.

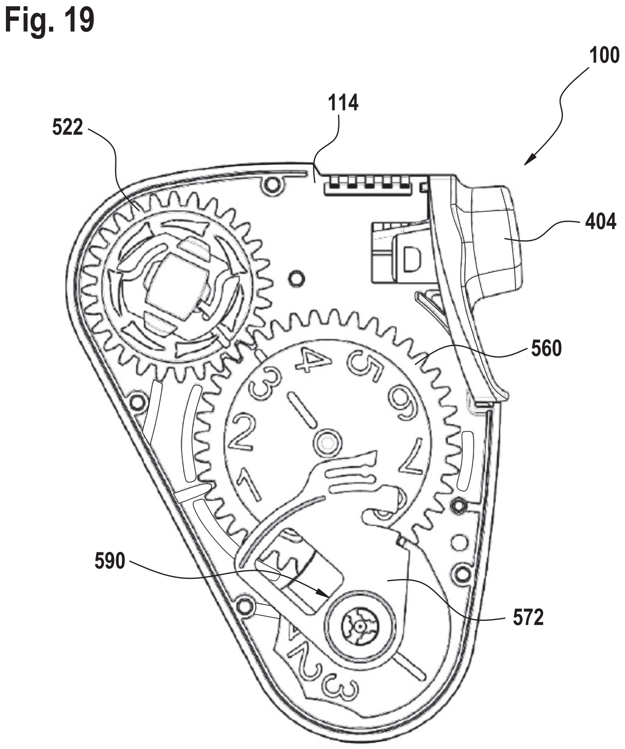

In Figures 18 and 19, a part of the flag 72 covering the dog 596 has been removed for illustrative purposes. In figure 19 the flag 72 covers the number 0 that can was visible through the window 590.

The engagement of the dog 596 with the tens disc 570 is triggered after the dose counter 580 displays 0, and further actuation beyond this point causes the tens disc 570 to turn and engage with the dog 596 to rotate the rotatable flag 572.

The dog 596 and the rotatable flag 570 are configured to rotate the flag 570 only after the final dose is expelled, ensuring that the dose counter 580 is obscured only after the last use of the device. In other words: To provide a clear indication to the user when all the pockets have been used the shutter or rotatable flag 570 is provided that blanks off the dose counter window or opening 590 when that point is reached.

In other words: For most of the rotation of the ones gear 560 the tens disc 570 is prevented from turning by its shaped outer profile locating on the ones gear 560. As the units change from zero to nine a semi circular recess in the ones gear disc allows the tens disc 570 to start turning by the influence of the friction drive. The cut out then engages with the tens disc 570 shaped profile and mechanically drives it to the next retained position. This process continues till the count reaches nine when the tens disc shows a coloured patch in place of a numeral to warn the user that there are only nine doses left. When the last pocket is reached the dose counter 580 shows zero. If the apparatus 100 is used beyond this point the tens disc 570 is again turned by the ones gear 560 and engages with the dog on the flag 570 causing the flag 570 to turn and obscure the dose counter with red shutter. A latch on the top face of the flag 570 locks it in this position.

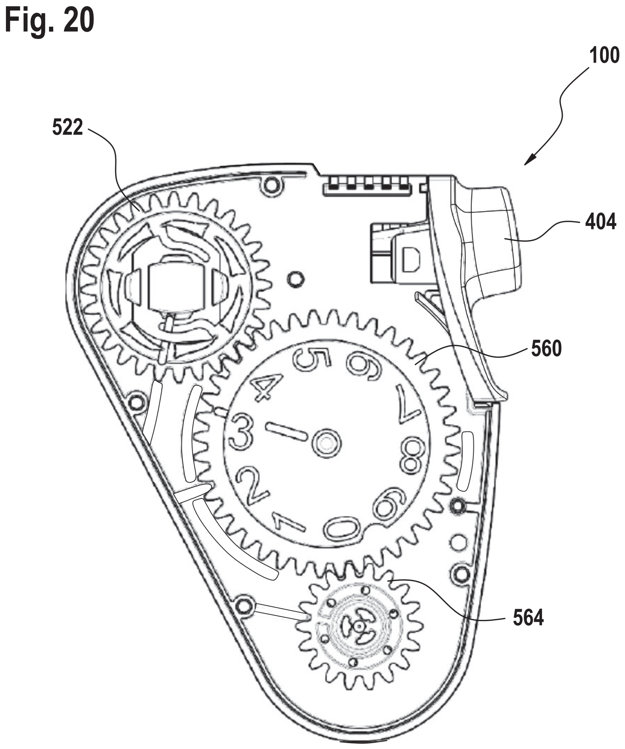

Figure 20 depicts the apparatus 100 with the flag 272 and the tens disc 570 removed. The does counter 580 further comprises a base winder gear 564 engaging with the ones gear 560 and configured to rotate one a defined degree of a turn for each actuation of the transporter gear 522, wherein the base winder gear 564 connected to a base winder shaft 552 that comprises at least one wind-up section 550a-b to wind-up a base foil.

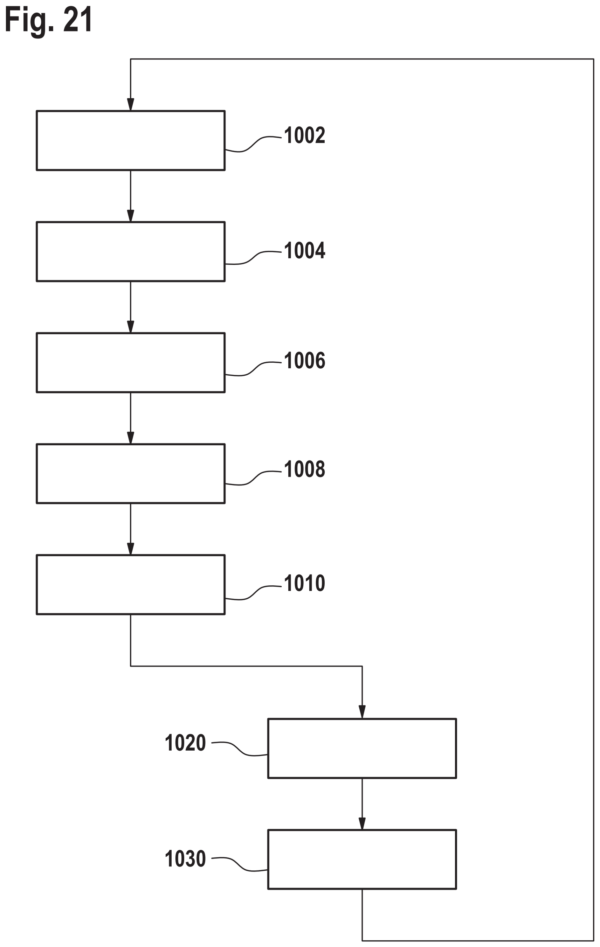

The base winder gear 564 has three segment projections that the tens disc 570 is located on. These provide a light friction drive to the tens disc as the gear turns. Figure 21 illustrates a schematic flow diagram depicting the steps 1002 to 1010 of the method for operating the apparatus 100 for administering at least one dose of a medicament to a patient. This method applies to any of the described apparatuses 100, including the one-strip models shown in Figures 13 and 14, as well as the two-strip models depicted in Figures 3, 5, 6, 7, 8, 11, and 12. For better understanding, the reference signs from the previous figures are used in the following.

At the beginning of the actuation cycle, the outer shape of the apparatus 100 is as shown in Figure 1. The apparatus 100 includes the cover 402, which functions as the manual lever. Initiating the rotation of the manual lever initiates the actuation cycle, as indicated in step 1002. The manual lever is rotated through an actuation angle below 90°, preferably between 60° and 75°, until it reaches a stop portion that defines the termination of its rotation, thereby terminating the actuation cycle. The termination of the actuation cycle is shown in Figure 2.

After the termination of the cycle, the manual lever is moved back to the position as shown in figure 1. During this movement, no force is coupled into the lid foil.

The at least one medicament carrier 220a-b comprises: a first portion comprising a plurality of closed pockets 202a#1-n, 202b#1-n containing at least a part of the at least one dose of the medicament, a second portion used to move the medicament carrier 220a-b, the second portion being rolled up on the transporter hub 504a-b driven by the manual lever, and a third portion that comprises, in a used state of the apparatus, at least one opened pocket.

In particular, the first portion comprises the lid foil and base foil bound together to provide closed pockets with powder therein. The second portion comprises the lid foil only after the peeling. The third portion comprises the base foil only after the peeling.

During step 1002, operating the manual lever drives the transporter hub 504a-b, applying a tensile force to the second portion of the medicament carrier 220a-b, which is rolled up on the transporter hub 504a-b. This tensile force moves one of the closed pockets 202a#1-n, 202b#1-n of the first portion of the medicament carrier 220a-b from an initial position in a direction opposite to the extension of the first portion.

In step 1004, the medicament carrier 220a-b is moved solely by means of the tensile force acting on the second portion. This movement causes the one closed pocket 202a#1-n, 202b#1-n of the first portion to be displaced from the initial position towards the peeling edge 410. The first portion comprises a plurality of closed pockets, each containing the nominal dose, formed by recesses in the base foil 220a-b and closed by the lid foil 210a-b.

As the actuation cycle progresses to step 1006, the one closed pocket passes beyond the peeling edge 410. The peeling edge 410 is configured such that, upon the closed pocket passing beyond it, a separation occurs to open the closed pocket. Specifically, the lid foil 210a-b separates from the base foil 220a-b at the peeling edge 410, with the separated lid foil becoming part of the second portion and being wound onto the transporter hub 504a-b. This movement continues to be driven solely by the tensile force on the second portion.

In step 1008, the now-opened pocket 202a#1-n, 202b#1-n, as part of the third portion comprising only the base foil 220a-b with opened pockets, is further advanced from the peeling edge 410 towards a target position at the collector 420. This advancement occurs only due to the tensile force acting on the second portion. After separation at the peeling edge 410, the third portion passes the collector channel 340a-b, as shown in Figure 7, in the direction of the collector window 425a-b. The collector channel 340a-b guides the opened pocket towards the collector window 425a-b, positioning it for inhalation by the patient. At least the side of the third portion that provides the opened pockets is guided or passes nearby at least one wall of the collector channel 340a-b.

Step 1010 involves terminating the actuation cycle by positioning the opened pocket at the target position. The manual lever reaches the stop portion that defines the termination of its rotation, completing the actuation cycle. All movements during the actuation cycle have been effected solely by the tensile force applied to the second portion via the manual lever and transporter hub 504a-b.

Throughout this process, the outer shape of the apparatus 100 transitions from the initial state shown in Figure 1 to the final state depicted in Figure 2. The manual lever or cover 402 rotates through an angle below 90°, specifically between 60° and 75°, to facilitate the movement of the medicament carrier 220a-b and the opening of the pocket.

Absence of Index Wheel and Sole Reliance on Tensile Force: Unlike prior art devices that utilise an index wheel or similar mechanisms to guide and position the medicament carrier and pockets, the present apparatus 100 does not employ such components. Instead, the medicament carrier 220a-b and the pockets are not guided by an index wheel. The movement, peeling, and positioning of the pockets are governed exclusively by the tensile force acting on the lid foil 210a-b.

This design simplifies the apparatus 100 by reducing the number of mechanical parts required for operation. The reliance solely on tensile force enhances the reliability of the device, minimising potential points of failure associated with additional guiding mechanisms. The absence of an index wheel allows for smoother operation and a more compact design. Furthermore, the medicament carrier experience less material stress due to counteracting forces effected by mechanisms including an index wheel.

Applicability to One-Strip and Two-Strip Models: The method described in Figure 21 is applicable to both one-strip models, such as those illustrated in Figures 13 and 14, and two-strip models, as shown in Figures 3, 5, 6, 7, 8, 11, and 12. In the one-strip models, a single medicament carrier 220a-b is utilised, whereas the two-strip models employ two medicament carriers operating in tandem. Regardless of the configuration, the actuation cycle operates under the same principles, with the tensile force applied to the second portion driving all movements of the respective medicament carrier.

### Detailed Steps of the Actuation Cycle:

Initiating the Actuation Cycle (Step 1002): The patient operates the manual lever, causing it to rotate through an angle below 90°, preferably between 60° and 75°. This rotation drives the transporter hub 504a-b, applying a tensile force to the lid foil 210a-b of the medicament carrier 220a-b. The tensile force initiates the movement of one closed pocket from the initial position towards the peeling edge 410.

Moving the Medicament Carrier (Step 1004): The medicament carrier 220a-b moves solely due to the tensile force acting on the lid foil 210a-b. The closed pocket is displaced from the initial position in a direction opposite to the extension of the first portion, advancing towards the peeling edge 410.

Passing the Peeling Edge and Opening the Pocket (Step 1006): As the closed pocket passes beyond the peeling edge 410, the lid foil 210a-b separates from the base foil 220a-b, resulting in the opening of the pocket. The separated lid foil is wound onto the transporter hub 504a-b, and this movement continues to be driven solely by the tensile force.

Advancing the Opened Pocket (Step 1008): The now-opened pocket, forming part of the third portion comprising only the base foil 220a-b, advances from the peeling edge 410 towards the collector window 425a-b through the collector channel 340a-b. This advancement occurs exclusively due to the tensile force acting on the lid foil 210a-b.

Terminating the Actuation Cycle (Step 1010): The actuation cycle is terminated when the manual lever reaches the stop portion that defines the end of its rotation. The opened pocket is positioned at the collector window 425a-b, ready for the patient to inhale the medicament-air mixture via the mouthpiece 404, which is in fluid connection with the collector window 425a-b.

The lid foil 210a-b and the base foil 220a-b are made of materials selected to ensure reliable separation at the peeling edge 410 when the tensile force is applied. The peeling edge 410 is configured to create a peeling angle sufficient to overcome the adhesion between the lid foil and the base foil at the closed pocket.

The manual actuation lever is mechanically linked to the transporter hub 504a-b to convert manual operation into rotational movement, thereby applying the tensile force to the lid foil 210a-b. The transporter hub 504a-b includes an attachment portion 506a-b for securing a leading portion of the lid foil. A foil rewind prevention mechanism 520 prevents unintended rewind of the lid foil, ensuring consistent operation.

The method depicted in Figure 21 demonstrates the simplified and efficient approach to operating the apparatus 100 for administering medicament doses. By relying solely on the tensile force applied to the lid foil 210a-b via the manual lever and transporter hub 504a-b, the apparatus eliminates the need for additional guiding mechanisms like index wheels. This design enhances reliability, reduces mechanical complexity, and ensures precise dosing with each actuation cycle.

The apparatus 100, as described, provides an effective solution for patients to administer medicament doses in powder form. The method ensures that each actuation cycle advances exactly one closed pocket from the initial position to the collector window 425a-b, maintaining accurate dosing and ease of use. The integration of the various components, including the manual lever, transporter hub, peeling edge, and collector channel, results in a cohesive system optimised for patient administration of medicaments.

Following the termination of the actuation cycle in step 1010, the method advances to step 1020. In step 1020, the opened pocket 202a#1-n, 202b#1-n remains positioned at the collector window 425a-b, precisely as achieved in step 1010. The medicament carrier 220a-b remains stationary in this position. At this stage, the patient can inhale the medicament-air mixture through the mouthpiece 404, which is in fluid connection with the collector window 425a-b and the opened pocket.

During inhalation, the patient places their lips around the mouthpiece 404 and inhales air. The airflow generated by the patient's inhalation passes through the opened pocket positioned over the collector window 425a-b. As air flows through the opened pocket, it entrains the medicament dose in powder form contained within the pocket. The medicament-air mixture is then drawn through the collector 420 and into the patient's respiratory system.

After the patient has completed inhalation in step 1020, the method proceeds to step 1030. In step 1030, the manual lever (cover 402) is returned to its initial position, as depicted in Figure 1. The patient or user rotates the manual lever back to its starting position, causing the cover 402 to close over the mouthpiece 404. This action serves multiple purposes: It resets the manual lever for the next actuation cycle. It protects the mouthpiece 404 from contamination or damage. It provides a visual indication that the apparatus 100 is ready for future use.

Importantly, during step 1030, the medicament carrier 220a-b remains in the position reached in step 1010 and maintained during step 1020. The opened pocket 202a#1-n, 202b#1-n remains over the collector window 425a-b, and the medicament carrier does not retract or move backward.

Step 1020: With the opened pocket in position over the collector window 425a-b, the patient inhales through the mouthpiece 404. Air flows through the opened pocket, entraining the medicament dose for inhalation. The medicament carrier 220a-b remains stationary in the position achieved in step 1010.

Step 1030: Following inhalation, the manual lever (cover 402) is returned to its start position, closing over the mouthpiece 404 as shown in Figure 1. This action resets the apparatus 100 for the next use while the medicament carrier 220a-b remains in the position reached during step 1010.

These steps 1020, 1030 complete the operational cycle of the apparatus 100. By allowing the medicament carrier 220a-b to remain stationary during inhalation, the apparatus ensures consistent and reliable delivery of the medicament dose. The patient benefits from a straightforward and intuitive process: initiate the actuation cycle, inhale the medicament-air mixture, and reset the apparatus by returning the manual lever to its starting position. Here the process may begin for the next inhalation cycle in step 1002.

The design emphasises simplicity and efficiency. The reliance solely on the tensile force applied to the lid foil 210a-b for movement, without the need for additional driving or braking mechanisms, reduces mechanical complexity and potential points of failure. The apparatus 100 is thus prepared for subsequent use, with the medicament carrier correctly positioned for the next actuation cycle.

In summary, step 1020 allows the patient to inhale the medicament-air mixture with the medicament carrier 220a-b remaining in the position reached in step 1010. Step 1030 involves returning the manual lever to its initial position, closing the cover 402 over the mouthpiece 404, and preparing the apparatus 100 for future use. The sequence of steps ensures efficient medicament delivery and maintains the readiness of the apparatus for administering subsequent doses.

## Claims

1. A collector (420) for an apparatus (100) for administering a medicament to a patient, the collector (420) comprising:
at least one conduit (440) extending through the collector (420) from at least one an entry section (442) toward a mouthpiece (404); wherein the entry section (442) of the conduit (440) comprises:
at least one collector window (425a-b) with at least one pocket airflow entry (426a-b) and at least one pocket airflow exit (424a-b), the pocket airflow entry (426a-b) configured to receive air to enter an opened pocket (202a#1-n, 202b#1-n) of an elongated medicament carrier (200a-b) arranged at the at least one collector window (425a-b), the at least one pocket airflow exit (424a-b) configured to receive a medicament-air mixture from the opened pocket (202a#1-n, 202b#1-n) to enter the at least one conduit (440); and
at least one bypass port (434a-b) configured to receive air to enter the at least one conduit (440), wherein the at least one bypass port (434a-b) and the at least one pocket airflow exit (424a-b) lead into opposing sides of the at least one conduit (440).

2. The collector (420) according to claim 1, wherein the at least one conduit (440) comprises at least one diffusor section (444a-b) along an air path from the entry section (442) towards the mouthpiece (404).

3. The collector (420) according to the claim 1, wherein the diameter increase of the at least one diffusor section is implemented along an imaginary plane (A-A) arranged between the at least one bypass port (434a-b) and at least one pocket airflow exit (424a-b).

4. The collector (420) according to claim 1 or 2, wherein the conduit (440) comprises a curvature or bend along its length, which defines an angle of less than 180° and more than 110°, in particular less than 180° and more than 150°, measured in a direction facing away from the at least one open pocket (202a#1-n, 202b#1-n).

5. The collector (420) according to one of the claims 1 to 4, wherein an open cross section of the at least one bypass port (434a-b) is equal or greater than an open cross section of the associated at least one pocket airflow entry (426a-b) or at least one pocket airflow exit (424a-b).

6. The collector (420) according to one of the claims 1 to 5, wherein a distance (D) between the at least one bypass port (434a-b) and the at least one pocket airflow exit (424a-b) is equal or greater than 1.4 multiplied with the equivalent diameter of an imaginary circular opening having the same cross-sectional area as the associated at least one pocket airflow exit (424a-b).

7. The collector (420) according to one of the claims 1 to 6, wherein the at least one bypass port (434a-b) and the at least one pocket airflow exit (424a-b) extend parallel to each other.

8. An apparatus (100) for administering a medicament to a patient comprising the collector (420) according to one of the claims 1 to 7.

9. The apparatus (100) according to claim 8, the apparatus (100) comprising:
the at least one elongated medicament carrier (200a-b), wherein the at least one medicament carrier (200a-b) comprises a plurality of closed pockets (202a#1-n, 202b#1-n) that extend along the at least one medicament carrier (200a-b), wherein the closed pockets (202a#1-n, 202b#1-n) contain a medicament in powder form, wherein the closed pockets (202a#1-n, 202b#1-n) are formed by recesses in a base foil (220a-b), and wherein a lid foil (216a-b) closes the plurality of openings of the recesses in the base foil (220a-b);
a peeling edge (410) configured, together with a manual actuation lever and a transporter hub (504a-b), to peel off the lid foil (210a-b) of the at least one medicament carrier (200a-b) from the base foil (220a-b) thereby opening the at least one of the plurality of closed pockets (202) in the base foil (220a-b);
the manual actuation lever;
the transporter hub (504a-b) that is operated by the manual actuation lever, wherein the transporter hub (504a-b) comprises an attachment portion (506a-b) for securing a leading portion (216a-b) of the lid foil (210a-b), and wherein the transporter hub (504a-b) is configured, together with the peeling edge (410), to move the opened pocket (202a#1-n, 202b#1-n) of the base foil (220a-b) toward the collector window (425a-b) solely via a tensile force coupled into the lid foil (210a-b) by means of the transporter hub (504a-b);
a foil rewind prevention mechanism (520) configured to prevent an unintended rewind of the lid foil (210a-b);
the collector window (425a-b), over which the respective opened pocket (202a#1-n, 202b#1-n) is moved; and
the mouthpiece (404) fluidly connected with the collector window (425a-b) by the conduit (440), wherein the mouthpiece (404) is configured to allow the patient to inhale a medicament-air mixture that comprises powder from the opened pocket (202a#1-n, 202b#1-n) arranged at the collector window (425a-b).
